# EUROPEAN PATENT SPECIFICATION

(11) **EP 2 279 002 B1**
(45) Date of publication and mention of the grant of the patent: **14.08.2013**
(21) Application number: 09738933.2
(22) Date of filing: 16.04.2009
(51) Int. Cl.: C07K 16/30, A61K 39/395, C12P 21/04, C12N 9/12, A61K 36/48, C07K 16/44, C12N 9/00, C12N 9/22

(54) **ANTI-NUCLEIC ACID ANTIBODY INDUCING CELL DEATH OF CANCER CELLS AND COMPOSITION FOR PREVENTING OR TREATING CANCERS COMPRISING THE SAME**
ANTI-NUKLEINSÄURE-ANTIKÖRPER ZUR INDUKTION VON ZELLTOD VON KREBSZELLEN UND DIESEN ENTHALTENDE ZUSAMMENSETZUNG ZUR PRÄVENTION ODER BEHANDLUNG VON KREBS
ANTICORPS ANTI-ACIDE NUCLÉIQUE INDUISANT LA MORT CELLULAIRE DE CELLULES CANCÉREUSES ET COMPOSITION POUR PRÉVENIR OU TRAITER DES CANCERS COMPRENANT CELUI-CI

(30) Priority: 30.04.2008 KR 20080040294
(43) Date of publication of application: 02.02.2011
(73) Proprietor: Ajou University Industry-Academic Cooperation Foundation, Suwon, Gyeonggi-do 443-749 (KR)
(72) Inventor: KWON, Myung-Hee, Suwon-si Gyeonggi-do 443-380 (KR); KIM, Yong-Sung, Suwon-si Gyeonggi-do 443-710 (KR); JANG, Ji-Young, Seoul 137-772 (KR)
(74) Representative: Tombling, Adrian George
(86) International application number: PCT/KR2009/001973
(87) International publication number: WO 2009/134027

(56) References cited:
- WO-A1-2008/035894
- US-A- 5 840 840
- US-A1- 2002 187 153
- US-A1- 2006 233 780
- US-B2- 6 653 104
- KOZYR A V ET AL: "Anti-DNA autoantibodies reveal toxicity to tumor cell lines.", IMMUNOLOGY LETTERS 1 JAN 2002 LNKD- PUBMED:11716964, vol. 80, no. 1, 1 January 2002 (2002-01-01), pages 41-47, XP002654584, ISSN: 0165-2478
- LEE ET AL: "Cell-penetrating autoantibody induces caspase-mediated apoptosis through catalytic hydrolysis of DNA", BIOORGANIC & MEDICINAL CHEMISTRY, PERGAMON, GB, vol. 15, no. 5, 31 January 2007 (2007-01-31), pages 2016-2023, XP005867169, ISSN: 0968-0896, DOI: DOI:10.1016/J.BMC.2006.12.037
- HANSEN JAMES E ET AL: "Antibody mediated transduction of therapeutic proteins into living cells.", THESCIENTIFICWORLDJOURNAL 16 SEP 2005 LNKD- PUBMED:16170440, vol. 5, 16 September 2005 (2005-09-16), pages 782-788, XP002654585, ISSN: 1537-744X
- KIM, Y.-R. ET AL.: 'Heavy and Light Chain Variable Single Domains of an Anti-DNA Binding Antibody Hydrolyze Both Double- and Single-stranded DNAs without Sequence Specificity' J. BIOL. CHEM. vol. 281, no. 22, 02 June 2006, pages 15287 - 15295, XP008108587
- NEVINSKY G. A. ET AL.: 'Human catalytic RNA- and DNA-hydrolyzing antibodies' J. IMMUNOL. METHODS vol. 269, no. 1-2, 01 November 2002, pages 235 - 249, XP004387969

## Description

### Technical Field

[1] The present invention relates to an anti-nucleic acid antibody inducing cell death of cancer cells by invading normal cells, and more particularly, to an anticancer effect of an anti-nucleic acid antibody that shows cytotoxicity by damaging nucleic acid strands, that is, genetic information of a cancer cell when the anti-nucleic acid antibody, which has binding activity and degrading activity to the nucleic acid strands in cells at the same time, is overexpressed in the cancer cell, or flows in the cancer cell.

[2] **Background Art**

[3] Recently, anti-nucleic acid antibodies have been reported to show their toxicities in cancer cells and to induce the cell death. First, it was reported that an anti-nucleic acid antibody isolated from sera of patients with systemic lupus erythematosus (SLE) and chronic lymphatic leukemia (CLL) show the cytotoxicity and induce the cell death (Immunology Letters. (2002) 80: 41-47. AV Kozyr, et al.). Second, it was reported that an anti-DNA antibody from patients with lupus erythematosus (LE) reaches the nucleus to induce the cleavage of poly (ADP-ribose) polymerase (PARP) which is a DNA repair enzyme (Journal of Clinical IImmunolgy. (2005) 25 : 99-105. Ingrid Bohm). Third, it was reported that an anti-DNA antibody (F4.1) permeates through cells to induce the cell death (Journal of Autoimmunity (2006) 26 : 52-56. Liliana Rivadeneyra-Espinoza, et al.). Finally, it was reported that an anti-DNA antibody (G1-5) obtained from MRL lpr/lpr mice uses a nuclease activity to induce the cell death (Bioorganic & Medical Chemistry (2007) 15 : 2016-2023. Eun-Jung Lee, et al.).

[4] Meanwhile, there is no patent registered, which discloses that the anti-nucleic acid antibody having nuclease activity directly shows the cytotoxicity in cancer cells, but there are some patents disclosing that the cytotoxicity is confirmed in the cancer cells by using enzymes having the ribonuclease activity.

[5] For example, US Patent No. 6,653,104 B2 discloses that an antibody associated with ribonuclease activity flows into acute tumoric cell and shows the toxicity to the acute tumoric cell.

[6] Also, US Patent No. 6 395,276 B 1 discloses that a *Rana pipiens* protein having the ribonucleolytic activity linked to an antibody capable of specific binding with a surface of a tumor cell kills cancer cells in selective and effective manners.

[7] Additionally, US Patent No. 6,869,604 B 1 discloses that recombinant ribonuclease proteins show the toxicity to the cancer cells when they are expressed by bacteria.

[8] Furthermore, US Patent No. 5,840,840 discloses selective RNase cytotoxic reagents prepared by linking ribonuclease to an antibody.

[9] As described above, there have been active attempts to develop therapeutic agents to treat cancers using the ribonucleolytic activity (BioDrugs. (2008) 22 : 53-58. Lee JE, et al.). Also, in order to maximize the cytotoxic effect (Protein Eng Des Sel. (2007) 20 : 505-509. Fuchs SM, et al.), and to minimize the side-effects, there is tendency toward the endowment of selectivity to the cancer cells (Biochem Biophys Res Commun. (2005) 331 : 595-602. Jurgen Krauss, et al.).

[10] However, the therapeutic agents to treat cancer cells using the above-mentioned ribonuclease activity have an advantage in that they may flow in the cancer cells by themselves, but has a problem in that the cytotoxic effect on the cancer cells is lowered by ribonuclease inhibitors that are present in an excessive amount in cytoplasm (J Biol Chem. (2004) 279 :39195-39198. Monti DM, et al.). On the contrary, the anti-nucleic acid antibody, 3D8 scFv, according to the present invention is expected to form the basis of development of more effective therapeutic agents to treat cancers since the 3D8 scFv may overcome the effect on ribonuclease inhibitors and have the ability to degrade deoxyribonucleic acids as well as ribonucleic acids.

[11] Meanwhile, it was reported that an extremely small number of the antibody proteins are refereed to as catalytic antibodies that show enzymatic activity to antigens while binging to the antigens, and anti-DNA, anti-RNA and anti-DNA/RNA antibodies have been reported as the endogenous catalytic antibodies (J. Cell. Mol. Med. (1998) Appl Biochem Biotechnol 75: 63-76, GA. Nevinsky, et al.; Cell Mol Life Sci (2003) 60:309-320 YJ. Jang, et al.)

[12] Also, BV04-01 was solely reported as the recombinant single-chain variable fragment (scFv) having the structural and catalytic characteristics as an anti-DNA catalytic antibody that degrades single-strand and double-strand DNA (Mol Immunol (1997) 34:1083-1093 Gololobov, G. V., et al.).

[13] However, no specific mechanism where these antibody proteins flow into cancer cells is reported in the art at all.

[14] Up to now, the mechanisms of therapeutic proteins using the antibody have been widely reported to inhibit the cell growth by binging to antigens present in cell membrane, or to directly induce the cell death. However, there is no case using an antibody with nuclease activity to treat or prevent cancers.

### Disclosure of Invention

### Technical Problem

[15] The present invention is designed to solve the problems of the prior art, and therefore it is an object of the present invention to provide a nuclease antibody having the ability to hydrolyze a variety of nucleic acids (DNA, RNA) regardless of any specific nucleotide sequences.

[16] Also, it is another object of the present invention to provide a composition for treating or preventing cancers comprising the nuclease antibody.

[17]

### Technical Solution

[18] According to the present invention, there is provided an antibody protein comprising: (i) a scFv having the sequence set forth in SEQ ID NO: 1; or (ii) a FV comprising a VH having the sequence set forth in SEQ ID NO: 2 and a VL having the sequence set forth in SEQ ID NO: 3, for use in preventing or treating cancer.

[24] In accordance with one exemplary embodiment of the present invention, the cancers may include cervix cancer, colon cancer, neuroblastoma, and liver cancer, but the present invention is not particularly limited thereto.

[25] The expression "nucleic acid" used in this specification preferably includes nucleic acids in cells, but the present invention is not particularly limited thereto.

[26] The expression" nuclease activity" used in this specification means the ability to cleave phosphoester bonds (3'- and 5'-phosphoester bonds) in deoxyribonucleic acid (DNA) and ribonucleic acid (RNA) strands through the hydrolysis reaction.

[27] Also, the term "cell" used in this specification preferably refers to cancer cells.

[28] The expression "cell permeating activity" used in this specification means an ability of a protein by itself to pass through an animal cell membrane and flow in cells without any artificial modification and handling of the protein and binding of the protein to other molecules.

[29] The anti-nucleic acid antibody used in the present invention is a 3D8 scFv (recombinant single-chain variable fragment) that has the ability to bind to and degrade DNA and RNA. Also, since the protein by itself invades cells to induce the cell death, the protein may be developed as a therapeutic agent to treat cancers.

[30] The anti-nucleic acid antibody according to one exemplary embodiment of the present invention has an activity to degrade nucleic acid strands in cells.

[31]

[32] Hereinafter, exemplary embodiments of the present invention will be described in detail.

[33] The present invention discloses that nuclease antibodies having the ability to hydrolyze a variety of nucleic acids (DNA, RNA) regardless of specific nucleotide sequences invade cancer cells. Here, when the nuclease antibodies having the ability flow into the cancer cells, the nuclease antibodies is cytotoxic to the cancer cells by causing the damage of nucleic acid strands, that is, genetic information, of the cancer cells, thereby showing an anticancer effect. Therefore, the antibodies having the nuclease activity and the cell-permeating activity at the same may be used to treat or prevent cancers.

[34] Meanwhile, the antibody protein having the nuclease activity and the cell-permeating activity at the same time according to the present invention preferably include immunoglobulin or fragments and/or derivatives thereof. Here, the fragments of the immunoglobulin having the nuclease activity and the cell-permeating activity at the same time may be used herein.

[35] The term "protein" used in the present invention means a polymer of amino acids. In this case, the term "antibody" described in the present invention is also included in the meanings of the protein. The term "fragment" used in the present invention refers to a polypeptide sequence having at least 20 consecutive amino acids or encoded by at least 50 consecutive amino acids. The term "derivatives" means a protein or fragments thereof, which undergo the substitution of at least one non-conservative or conservative amino acid in a polypeptide, or the modification of the second molecule by a covalent bonding, such as, for example, adhesion or glycosylation, acetylation, phosphorylation of heterologous peptides.

[36] The term "prevent," "preventing" and "prevention" used in the present invention refers to the administration of an antibody or derivatives thereof according to one exemplary embodiment of the present invention into an individual before the individual ultimately manifests at least diagnostic or clinical condition of a cancer in order to suppress the onset or development of the cancer, inhibit the onset of the cancer or relieve the symptom of the onset of the cancer.

[37] The term "treat," "treating" and "treatment" used in the present invention refers to the administration of an antibody or derivatives thereof according to one exemplary embodiment of the present invention into an individual after the individual ultimately manifests at least diagnostic or clinical condition of a cancer at any clinical phase in order to suppress the onset and development of the symptom of the cancer inhibit the onset of the cancer or relieve the symptom of the onset of the cancer, thereby reducing or getting rid of the clinical or diagnostic conditions of the cancer to relieve and/or stop the patients cancer. The treatment may include the reduction in the severity and number of the symptoms of the cancer and the recurrence of the cancer.

[38] The expression "pharmaceutically available component" used in the present invention refers to a vehicle, a diluent, an adjuvant or an additive with which antibody is administered.

[39] The expression "effective amount" used in the present invention refers to an amount of an antibody or derivatives thereof that is sufficient to relieve at least one clinical or diagnostic condition of cancer at patients or inhibit the onset of the cancer. Medicines are administered according to their "regimens for administration and prescription." The expression "regimens for administration and prescription" means the combination of dosage and dosing frequency of drugs, which are suitable for achieving the treatment or prevention of cancers.

[40] In accordance with some exemplary embodiment, the antibody of the present invention may be a chimeric antibody. The expression "chimeric antibody" means an antibody having various regions derived from animal species other than a human, for example an antibody having a variable region derived from a murine monoclonal antibody and a human IgG immunoglobulin immobilization region. The method for producing a chimeric antibody was known in the art (for example, see Morrison, Science, 1985, 229:1202; Oi et al., 1986, BioTechniques 4:214; Gillies et al., 1989, J. Immunol. Methods 125:191-202; US Patent Nos: 5,807,715; 4,816,567; and 4,816,397).

[41] The antibody according to one exemplary embodiment of the present invention may be prepared according to one of the methods widely known in the art. For example, the monoclonal antibodies may be prepared in a wide variety of techniques including hybridoma, recombinant and phage display techniques or combinations thereof. The hybridoma techniques are generally disclosed, for example, in Harlow et al., Antibodies: A Laboratory Manual (Cold Spring Harbor Laboratory Press, 2nd ed., 1988); and Hammerling, et al., In Monoclonal Antibodies and T-Cell Hybridomas, pp. 563-681 (Elsevier, N.Y., 1981). The phage display techniques are disclosed, for example, in various documents (Hoogenboom and Winter, 1991, J. Mol. Biol. 227:381; Marks et al., 1991, J. Mol. Biol. 222:581; Quan and Carter, 2002, The rise of monoclonal antibodies as therapeutics in Anti-IgE and Allergic Disease, Jardieu and Fick Jr., eds., Marcel Dekker, New York, N.Y., Chapter 20, pp. 427-469; Brinkman et al., 1995, J. Immunol. Methods 182:41-50; Ames et al., 1995, J. Immunol. Methods 184:177-186; Kettleborough et al., 1994, Eur. J. Immunol 24:952-958; Persic et al., 1997, Gene 187:9-18; Burton et al., 1994, Advances in Immunology 57:191-280; PCT Application No. PCT/GB91/01134; PCT WO 90/02809, WO 91/10737, WO 92/01047, WO 92/18619, WO 93/11236, WO 95/15982, WO 95/20401, and US Patent Nos: 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108).

[42] The techniques used to produce single-chain antibodies are disclosed, for example, in US Patent Nos: 4,946,778 and 5,258,498; Huston et al., 1991, Methods in Enzymology 203:46-88; Shu et al., 1993, Proc. Natl. Acad. Sci. USA 90:7995-7999; and Skerra et al., 1988, Science 240:1038-1040.

[43] Methods for making bispecific antibodies are known in the art. Conventional production of full-length bispecific antibodies is based on the co-expression on two immunoglobulin heavy chain-light chain pairs, where the two chains have different specificities (see Milstein et al., 1983, Nature 305:537-39). Because of the random assortment of immunoglobulin heavy and light chains, these hybridomas produce a potential mixture of 10 different antibody molecules, of which only one has the correct bispecific structure. Similar procedures are disclosed in WO 93/08829.

[44] For the further information about the bispecific antibodies, for example, see Suresh et al., 1986, Methods in Enzymology 121:210; Rodrigues et al., 1993, J. Immunology 151:6954-61; Carter et al., 1992, Bio/Technology 10:163-67; Carteret al., 1995, J. Hematotherapy 4:463-70; Merchant et al., 1998, Nature Biotechnology 16:677-81.

[45] The antibody of the present invention may be a humanized antibody. Humanized antibodies are antibody molecules that have a backbone and an immobilization region from a human immunoglobulin molecule; and one or more CDRs from non-human species and bind to desired antigens. Often, backbone residues in human backbone regions may be substituted with corresponding residues derived from a CDR donor antibody in order to improve the binding to antigens. These backbone substitutions are identified according to one of the methods known in the art (for example, Queen et al., US Patent No. 5,585,089; Riechmann et al., 1988, Nature 332:323.) Antibodies may be humanized using a variety of techniques known in the art, for example CDR-grafting (for example, see EP 0 239 400; PCT WO 91/09967; US Patent Nos. 5,225,539; 5,530,101; and 5,585,089), veneering or resurfacing (for example EP 0 592 106; EP 0 519 596; Padlan, Molecular Immunology, 1991, 28(4/5):489-498; Studnicka et al., 1994, Protein Engineering 7(6):805-814; Roguska et al., 1994, Proc. Natl. Acad. Sci. USA 91:969-973), and chain shuffling (for example, see US Patent No. 5,565,332).

[46] Humanized monoclonal antibodies may be produced according to one of the recombinant DNA techniques using, for example, the methods disclosed in PCT WO 87/02671; European Patent Publication Nos. 0 184 187; 0 171 496; and 0 173 494; PCT WO 86/01533, etc.

[47] In accordance with some exemplary embodiment, the antibody of the present invention is a mouse IgG antibody. Mouse antibodies may be, for example, prepared according to a variety of the prior-art techniques including phage display methods using an antibody library derived from human immunoglobulin sequences. For example, see US Patent Nos. 4,444,887 and 4,716,111; and PCT WO 98/46645, WO 98/50433, WO 98/24893, WO 98/16654, WO 96/34096, WO 96/33735, and WO 91/10741.

[48] The antibody and derivatives thereof according to one exemplary embodiment of the present invention are produced by the prior-art methods associated with the protein synthesis, for example a recombinant expression technique. For the purpose of the recombinant expression of the antibody and derivatives thereof according to one exemplary embodiment of the present invention, it is necessary to construct an expression vector containing nucleic acids encoding the antibody and derivatives thereof according to one exemplary embodiment of the present invention. The expression vector may be constructed according to the recombinant DNA technologies using the techniques known in the art. These standard techniques may include, for example, a recombinant nucleic acid method, nucleic acid synthesis, cell culture, transgene insertion and a recombinant protein expression technique, as disclosed in Sambrook and Russell, Molecular Cloning: A Laboratory Manual (Cold Spring Harbor Laboratory Press, Cold Spring Harbor, N.Y., 3rd ed., 2001); Sambrook et al., Molecular Cloning: A Laboratory Manual (Cold Spring Harbor Laboratory Press, Cold Spring Harbor, N.Y., 2nd ed., 1989); Short Protocols in Molecular Biology (Ausubel et al., John Wiley and Sons, New York, 4th ed., 1999); and Glick and Pasternak, Molecular Biotechnology: Principles and Applications of Recombinant DNA (ASM Press, Washington, D.C., 2nd ed., 1998).

[49] In order to express the antibody of the present invention in a recombinant manner, for example, an expression vector encoding a heavy chain or light chain may be functionally linked to a promoter. For example, the expression vector may have a nucleotide sequence encoding an immobilization region of an antibody molecule (for example, PCT WO 86/05807; WO 89/01036; and US Patent No. 5,122,464), and a variable region of the antibody may be cloned into a vector used to express full-length heavy and light chains of the antibody. The expression vector is transfected according to the conventional techniques, and cultured according to the conventional method for producing antibody. In accordance with a typical embodiment of the expression of double-strand antibodies, vectors encoding all the heavy and light chains may be co-expressed in a host cell that is used to express a full-length immunoglobulin molecule.

[50] A variety of prokaryotic and eukaryotic host expression vector systems may be used to express the antibody or derivatives thereof according to one exemplary embodiment of the present invention. Typically, eukaryotic cells for the full-length recombinant antibody molecules may be used to expressing a recombinant protein. For example, mammalian cells such as CHO cell may be an effective expression system to produce the antibody according to one exemplary embodiment of the present invention, as in the vector containing an early gene promoter derived from human CMV virus (see Foecking et al., 1986, Gene 45:101; Cockett et al., 1990, Bio/Technology 8:2).

[51] Other host-expression systems include, for example, a plasmid-based expression system in bacterial cell (for example, Ruther et al., 1983, EMBO 1, 2:1791; Inouye and Inouye, 1985, Nucleic Acids Res. 13:3101-3109; Van Heeke and Schuster, 1989, J. Biol. Chem. 24:5503-5509); an insect system; and a virus-based expression system in mammalian cell, for example, an adenovirus-based system (for example, Logan and Shenk, 1984, Proc. Natl. Acad. Sci. USA 81:355-359; Bittner et al., 1987, Methods in Enzymol. 153:51-544).

[52] Also, a host cell may be selected to control the expression of inserted sequences, or modify or process desired gene products. Suitable cell lines or host systems may be selected for the purpose of the exact modification and processing of expressed proteins. These mammalian host cells include, for example, CHO, VERO, BHK, HeLa, COS, MDCK, 293, 3T3, and W138, but the present invention is not particularly limited thereto.

[53] An expression level of the antibody according to the present invention may be increased by vector amplification (for example, see Bebbington and Hentschel, The Use of Vectors Based on Gene Amplification for the Expression of Cloned Genes in Mammalian Cells in DNA Cloning, Vol. 3 (Academic Press, New York, 1987)).

[54] When the antibody according to the present invention includes all the heavy and light chains or derivatives thereof, a host cell may be co-transfected with two expression vectors: one vector encoding a heavy chain protein and the other encoding a light chain protein.

[55] Once the antibody of the present invention is produced, the produced antibody may be purified using suitable methods such as chromatography, etc.

[56] A composition comprising the antibody or derivatives thereof according to one exemplary embodiment of the present invention may be administered to an object who suffers from cancer, or is at risk of developing into cancer. The term "object" used herein means mammalian patients, to which the antibody of the present invention may be administered, including human and non-human Mammalia. More preferably, an object that is treated with the antibody of the present invention refers to human beings.

[57] An amount of the antibody that is effective for the prevention and treatment of cancers may be determined according to the standard clinical techniques. Also, the *in-vitro* analysis may be selectively adopted in order to help to elucidate the optimum dosage range. An exact dose used in the formulation is dependent on the route of administration and the severity of cancer, and should be determined according to the physician's judgement and the patients' status. The effective amount may be calculated from a dose-dependent curve from the animal model or the *in-vitro* system.

[58] For example, toxicity and therapeutic effects of an antibody may be determined from the cell culture or test animal according to the standard pharmaceutical procedure, which is used to determine LD₅₀ and ED₅₀. A volume ratio between the toxicity and the therapeutic effects is represented by a therapeutic index, and expressed by a LD₅₀/ED₅₀ ratio.

[59] In general, an amount of the antibody of the present invention that is administered to cancer patients is at a dose of 0.1 to 100 mg/kg body weight daily. An amount of the antibody that is administered to patients is in a range of 0.1 to 50 mg/kg, preferably 0.1 to 20 mg/kg body weight. In general, human antibodies have a longer half-life in human body than other species-derived antibodies due to the immune response to exogenous proteins. Therefore, antibodies including humanized chimeric or human antibodies may be administered at a smaller dose and at a lower dosing frequency.

[60] The term "antibody" used in the present invention refers to (a) an immunoglobulin protein and immunologically active regions of the immunoglobulin protein (i.e. an immunoglobulin-related protein and its fragments including an antigen-binding site that immune-specifically binds to specific antigens), or (b) conservatively substituted derivatives of the immunoglobulin protein or its fragments that immune-specifically bind to antigens. In general, the antibodies are disclosed, for example, in Harlow and Lane, Antibodies: A Laboratory Manual (Cold Spring Harbor Laboratory Press, 1988).

[61] The expression "conservative substitution" used in the present invention refers to the substitution of at least one amino acid, which does not substantially reduce a specific binding affinity of the immunoglobulin protein or its fragments to the antigens.

[62] The expression "antibody derivatives" used in the present invention means antibodies modified by covalent bonding of heterogeneous molecules (binding glycosylation and acetylation of heterogeneous peptides).

[63] In accordance with the present invention, the immunoglobulin is preferably an immunoglobulin G, and the immunoglobulin G is more preferably a 3D8 protein.

[64] The term "immunoglobulin" is the general term for proteins that play an important role in immunity in serum components and act as antibodies. The immunoglobulin has a basic structure where a pair of L chains (light chain) each having a molecular weight of approximately 2, 3000 bind to a pair of H chains (heavy chain) each having a molecular weight of 50,000 to 70,000 through disulfide bonds. Here, the immunoglobulins are divided into IgG, IgA, IgM, IgD and IgE, depending on the kinds (γ,α,µ,δ and ε of the H chains.

[65] The immunoglobulin G (IgG) is composed of a pair of L chains each having a molecular weight of approximately 2,5000 and a pair of H chains each having a molecular weight of approximately 50,000, and thus has the total molecular weight of approximately 150,000. Also, the IgG is one of the important immunoglobulins in higher animals such as amphibian or higher animals, and accounts for approximately 70% of the immunoglobulins in human beings, is transported across the placenta, and has various antibody activities.

[66]

[67] The single chain variable fragment (scFv) has a heavy chain variable region (VH) and a light chain variable region (VL) connected to each other by a linker, and it has been known that it conventionally has an excellent binding affinity to antigens, compared to the use of either the heavy chain variable region or the light chain variable region. In this case, when any linkers are conventionally used in the art, the linkers may be used as the linker connecting the VH and VL.

[68] The VH and VL have been known to have a lower binding affinity to antigens than the scFv, but it may be preferred to use the scFv for the purpose of improving the permeation into tissues, when necessary.

[69] The anti-cancer composition according to one exemplary embodiment of the present invention is administered in connection with therapies that have been conventionally used to heal, prevent or treat cancers. Examples of these conventional treatment methods include surgical operations, chemotherapy, radiation therapy, hormone therapy, biological therapy and immunotherapy, but the present invention is not particularly limited thereto.

[70] The term "cancer" used in this specification include solid tumors and blood-born tumors, but the present invention is not particularly limited thereto. Also, the "cancer" refers to disorders in skin tissues, organs, blood and blood vessels, including, but is not particularly limited to, cancers in bladder, bone or blood, brain, breast, cervix, chest, colon, endrometrium, esophagus, eyes, head, kidney, liver, lymph node, lungs, mouth, neck, ovary, pancreas, prostate gland, rectum, stomach, testicles, throat and uterus. The certain cancers include advanced malignancy, amyloidosis, neuroblastoma, meningioma, hemangiopericytoma, multiple brain metastase, glioblastoma multiforms, glioblastoma, brain stem glioma, poor prognosis malignant brain tumor, malignant glioma, recurrent malignant glioma, anaplastic astrocytoma, anaplastic oligoden-droglioma, neuroendocrine tumor, adenocarcinoma, Dukes C & D colorectal cancer, unresectable colorectal cancer, metastatic hepatocellular carcinoma, Kaposi's sarcoma, karyotype acute myeloid leukemia, Hodgkin's lymphoma, non-Hodgkin's lymphoma, cutanrous T-cell lymphoma, cutanrous B-cell lymphoma, diffuse large B-cell lymphoma, low-grade follicular lymphoma, malignant melanoma, malignant mesothelioma, malignant pleural effusion mesothelioma syndrome, appendiceal sarcoma, papillary serous carcinoma, gynecologic sarcoma, soft tissue sarcoma, scelroderma, subcutaneous vasculitis, Langerhan's cell histiocytosis, leiomyosarcoma, fibrodysplasia ossificans progressive, hormone-resistance prostate cancer, resected high-risk soft tissue sarcoma, unrescectable hepatocellular carcinoma, Waldenstrom's macroglobulinemia, smoldering myeloma, indolent myeloma, salpingitis, androgen-independent prostatic cancer, androgen-independent stage IV non-metastatic prostatic cancer, hormone-insensitive prostatic cancer, chemotherapy-insensitive prostatic cancer, papillary thyroid carcinoma, follicular thyroid carcinoma, medullary thyroid carcinoma, and leiomyoma, head and neck cancer, but the present invention is not particularly limited thereto.

[71] In accordance with the present invention, the cancers to be treated by the composition include cervix cancer, colon cancer, or neuroblastoma, but the present invention is not particularly limited thereto.

[72] In accordance with a specific embodiment, the cancers are metastatic. In accordance with another embodiment, the cancers are characterized in that they are intractable or resistant to chemotherapy or radiation therapy.

[73] The present invention encompasses a method for treating patients who were not previously treated for disorders or troubles associated with cancers or characterized by the cancers, or who were previously treated but had no response to standard therapy. Also, the present invention encompasses a method for treating patients regardless of the patients ages although some disorders or troubles are more prevalent in a certain age group. The present invention encompasses a method for treating patients who have had a surgical operation(s) for diseases or diseases that may or may not be problematic to the patient. Since a patient who suffers from the disorders or troubles characterized by the cancers has non-uniform clinical findings and various clinical results, the treatments in the patient may be varied according to his/her prognosis. Trained clinicians can easily determine certain secondary drugs, types of surgical operations and non-pharmacologic standard therapies, which may be effectively used to treat individual patients for cancers without any unreasonable experiments.

[74] Pharmaceutical compositions and dosage forms according to the present invention may also include at least one additional active component. As a result, the pharmaceutical compositions and dosage forms according to the present invention include an active component disclosed in this specification.

[75] A single-unit dosage form of the present invention is suitable for being administered to patients in an oral, mucosal (for example, intranasal, sublingual, vaginal, buccal, or rectal), parenteral (for example, subcutaneous, intravenous, bolus-injected, intramuscular or intraarterial), topical (for example, eyes), transdermal or transcutaneous manner.

[76] Examples of the dosage forms include liquid dose formulations that are suitable for being orally or mucosally administered to patients, comprising tablet, caplets, capsules (i.e. soft elastic gelatin capsule), cachets, troches, lozenges, dispersing agents, suppositories, powders, aerosols (for example, nasal spray or inhaler); gels, suspensions (for example, aqueous or non-aqueous liquid suspensions, oil-in-water emulsion, or water-in-oil liquid emulsion), solutions and elixirs; eye drop or other ophthalmic formulations that are suitable for being topically administered to patients; and sterile solid formulations (for example, crysalline or amorphous solids) that may be reconstituted to provide a liquid dosage form that is suitable for being injected to patients, but the present invention is not particularly limited thereto.

[77] Compositions, shapes, and types of the dosage forms of the present invention are widely varied according to their use. For example, a dosage form used to treat acute diseases may contain a higher amount of at least one active component than that of a dosage form used to treat the same chronic diseases. Similarly, a parenteral dosage form may contain a smaller amount of at least one active component than that of an oral dosage form used to treat the same diseases. These and other methods for certain dosage forms encompassed in the scope of the present invention are widely varied, and evident to those skilled in the art to which the present invention belongs. For example, see Remington's Pharmaceutical Sciences, 18th ed., Mack Publishing, Easton PA (1990).

[78] Typical pharmaceutical compositions and their dosage forms comprise at least one vehicle. Suitable vehicles are well-known to those skilled in the art of pharmacy, and non-limiting examples of the suitable vehicles are disclosed in this specification.

[79] Whether a particular vehicle is suitable for incorporation into a pharmaceutical composition or dosage form depends on a variety of factors well-known in the art including, but not limited to, the way in which the dosage form will be administered to a patient. For example, a pharmaceutical composition or dosage form may include a vehicle (i.e., tablet) that is not suitable for the use in the orally and parenteral dosage forms.

[80] The present invention further encompasses pharmaceutical compositions and dosage forms that comprise one or more compounds that reduce the rate by which an active component will decompose. Such compounds, which are referred to herein as "stabilizers," include, but are not particularly limited to, antioxidants such as ascorbic acid, pH buffers, or salt buffers.

[81] As in the amount and types of vehicles, amount and certain shapes of the active component in the dosage forms may depend on factors including, but is not particularly limited to, the route by which the active component is administered to a patient.

[82] Pharmaceutical compositions of the present invention that are suitable for oral administration may be presented as discrete dosage forms, such as, but are not limited to, tablets (e.g., chewable tablets), caplets, capsules, and liquids (e.g., flavored syrups). Such dosage forms contain predetermined amounts of active components, and may be prepared by methods of pharmacy well known to those skilled in the art to which the present invention belongs.. See generally, Remington's Pharmaceutical Sciences, 18th ed., Mack Publishing, Easton PA (1990).

[83] The generic oral dosage forms of the present invention are prepared by combining the active component(s) in an intimate admixture with at least one vehicle according to conventional pharmaceutical compounding techniques. Vehicles may take a wide variety of forms depending on the form of preparation desired for administration. For example, vehicles suitable for use in oral liquid or aerosol dosage forms include, but are not limited to, water, glycols, oils, alcohols, flavoring agents, preservatives, and coloring agents. Examples of vehicles suitable for use in solid oral dosage forms (e.g., powders, tablets, capsules, and caplets) include, but are not limited to, starches, sugars, microcrystalline cellulose, diluents, granulating agents, lubricants, binders, and disintegrants.

[84] Because of their ease of administration, tablets and capsules represent the most advantageous oral dosage unit forms, in which case solid vehicles are employed. If desired, tablets can be coated by standard aqueous or non-aqueous techniques. Such dosage forms can be prepared by any of the methods of pharmacy. In general, pharmaceutical compositions and dosage forms are prepared by uniformly and intimately mixing the active components with liquid carriers, finely divided solid carriers, or both, and then shaping the product into the desired presentation if necessary.

[85] Examples of vehicles that may be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatine, natural and synthetic gums such as acacia, sodium alginate, alginic acid, other alginates, powdered tragacanth, guar gum, cellulose and its derivatives (e.g., ethyl cellulose, cellulose acetate, carboxymethyl cellulose calcium, sodium carboxymethyl cellulose), polyvinyl pyrrolidone, methyl cellulose, pre gelatinized starch, hydroxypropyl methyl cellulose, (e.g., Nos. 2208, 2906, 2910), microcrystalline cellulose, and mixtures thereof.

[86] Suitable forms of microcrystalline cellulose include, but are not limited to, the materials sold as AVICEL PH 101, AVICEL PH 103 AVICEL RC 581, AVICEL PH 105 (available from FMC Corporation, American Viscose Division, Avicel Sales, Marcus Hook, Pa.), and mixtures thereof. A specific binder is a mixture of microcrystalline cellulose and sodium carboxymethyl cellulose sold as AVICEL RC 581. Suitable anhydrous or low-moisture vehicles or additives include AVICEL PH 103™ and Starch 1500 LM.

[87] Examples of fillers suitable for use in the pharmaceutical compositions and dosage forms disclosed in this specification include, but are not limited to, talc, calcium carbonate (e.g., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the present invention is typically present in from approximately 50 to approximately 99 % by weight of the pharmaceutical composition or dosage form.

[88] Disintegrants are used in the compositions of the invention to provide tablets that disintegrate when the tablets are exposed to an aqueous environment. Tablets that contain too much disintegrant may disintegrate in storage, while tablets that contain too little disintegrant may not disintegrate at a desired rate or under the desired conditions. Thus, a sufficient amount of disintegrant that is neither too much nor too little to detrimentally alter the release of the active components should be used to form solid oral dosage forms of the present invention. The amount of the used disintegrant varies based upon the type of formulation, and is readily discernible to those of ordinary skill in the art to which the present invention belongs. Typical pharmaceutical compositions comprise from approximately 0.5 to approximately 15 % by weight of disintegrant, preferably from approximately 1 to approximately 5 % by weight of disintegrant.

[89] Disintegrants that can be used in pharmaceutical compositions and dosage forms of the present inventioninclude, but are not limited to, agar agar, alginic acid, calcium carbonate, microcrystalline cellulose, croscarmellose sodium, crospovidone, polacrilin potassium, sodium starch glycolate, potato or tapioca starch, pre-gelatinized starch, other starches, clays, other algins, other celluloses, gums, and mixtures thereof.

[90] Lubricants that can be used in pharmaceutical compositions and dosage forms of the present invention include, but are not limited to, calcium stearate, magnesium stearate, mineral oil, light mineral oil, glycerine, sorbitol, mannitol, polyethylene glycol, other glycols, stearic acid, sodium lauryl sulfate, talc, hydrogenated vegetable oil (e.g., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, Md.), a coagulated aerosol of synthetic silica (marketed by Degussa Co. of Plano, Tex.), CAB-O-SIL (a pyrogenic silicon dioxide product sold by Cabot Co. of Boston, Mass.), and mixtures thereof. If used at all, lubricants are typically used in an amount of less than approximately 1 % by weight of the pharmaceutical compositions or dosage forms into which they are incorporated.

[91] Preferred oral dosage forms of the present invention include, but are not particularly limited to, selective cytokine inhibitors of the present invention, anhydrous lactose, microcrystalline cellulose, polyvinylpyrrolidone, stearic acid, colloidal amorphous silica, and gelatine.

[92] Active components of the present invention may be administered by controlled release systems or by delivery devices that are well known to those of ordinary skill in the art to which the present invention belongs. Examples include, but are not limited to, those described in U.S. Pat. Nos. 3,845,770; 3,916,899; 3,536,809; 3,598,123; and 4,008,719, 5,674,533, 5,059,595, 5,591,767, 5,120,548, 5,073,543, 5,639;476, 5,354,556, and 5,733,566. Such dosage forms may be used to provide slow or controlled release of one or more active components using, for example, hydropropylmethyl cellulose, other polymer matrices, gels, permeable membranes, osmotic systems, multi-layer coatings, microparticles, liposomes, microspheres, or a combination thereof to provide the desired release profile in varying proportions. Suitable controlled release formulations known to those of ordinary skill in the art to which the present invention belongs, including those described herein, may be readily selected for use with the active components of the present invention. The present invention thus encompasses single unit dosage forms suitable for oral administration such as, but not limited to, tablets, capsules, gelcaps, and caplets that are adapted for controlled release.

[93] All controlled-release pharmaceutical compositions have a common goal of improving drug therapeutic effects over that achieved by their non-controlled counterparts. Ideally, the use of an optimally designed controlled-release formulation in medical treatment is characterized by a minimum of drug substance being employed to treat or control the condition in a minimum amount of time. Advantages of controlled-release formulations include extended activity of the drug, reduced dosage frequency, and increased subject compliance. In addition, controlled-release formulations may be used to affect the time of onset of action or other characteristics, such as blood levels of the drug, and may thus affect the occurrence of side (e.g., adverse) effects.

[94] Most controlled-release formulations are designed to initially release an amount of drug (active component) that promptly produces the desired therapeutic effects, and gradually and continually release of other amounts of drug to maintain this level of therapeutic or prophylactic effects over an extended period of time. In order to maintain this constant level of drug in the body, the drug should be released from the dosage form at a rate that will replace the amount of drug being metabolized and excreted from the body. Controlled release of an active component may be stimulated by various conditions including, but not limited to, pH, temperature, enzymes, moisture, or other physiological conditions or compounds.

[95] Parenteral dosage forms may be administered to patients by various routes including, but not limited to, subcutaneous, intravenous (including bolus injection), intramuscular, and intraarterial routes. Because their administration typically bypasses patients' natural defenses against contaminants, parenteral dosage forms are preferably sterile or capable of being sterilized prior to administration to a spatient. Examples of the parenteral dosage forms include, but are not limited to, solutions ready for injection, dry products ready to be dissolved or suspended in a pharmaceutically acceptable vehicle for injection, suspensions ready for injection, and emulsions.

[96] Suitable vehicles that may be used to provide parenteral dosage forms of the present invention are well-known to those skilled in the art to which the present invention belongs. Examples include, but are not limited to: water for injection USP; aqueous vehicles such as, but not limited to, sodium chloride injection, Ringer's injection, dextrose injection, dextrose and sodium chloride injection, and lactated Ringer's injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

[97] Compounds that can increase the solubility of one or more of the active ingredients disclosed herein may also be incorporated into the parenteral dosage forms of the invention. For example, cyclodextrin and its derivatives may be used to increase the solubility of the selective cytokine inhibitors and their derivatives of the present invention. For example, see US Patent No. 5,134,127.

[98] Transdermal, topical, and mucosal dosage forms of the present invention include, but are not limited to, sprays, aerosols, solutions, emulsions, suspensions, eye drops, ophthalmic solutions, or other forms known to those skilled in the art to which the present invention belongs. See, e.g., Remington's Pharmaceutical Sciences, 16th, 18th eds., Mack Publishing, Easton Pa. (1980 & 1990); and Introduction to Pharmaceutical Dosage Forms, 4th ed., Lea & Febiger, Philadelphia (1985). Dosage forms suitable for treating mucosal tissues within the oral cavity may be formulated as mouthwashes or as oral gels.

[99] Suitable additives (e.g., carriers and diluents) and other materials that may be used to provide topical and mucosal dosage forms encompassed by the present invention are well known to those skilled in the pharmaceutical arts, and depend on the particular tissue to which a given pharmaceutical composition or dosage form will be applied. With that fact in mind, typical additives include, but are not limited to, water, acetone, ethanol, ethylene glycol, propylene glycol, butane-1,3-diol, isopropyl myristate, isopropyl palmitate, mineral oil, and mixtures thereof to form solutions, emulsions, or gels, which are non-toxic and pharmaceutically acceptable. Moisturizers or humectants may also be added to pharmaceutical compositions and dosage forms if desired. Examples of such additional ingredients are well-known to those skilled in the art to which the present invention belongs. See, e.g., Remington's Pharmaceutical Sciences, 16th , and 18th eds., Mack Publishing, Easton Pa. (1980 & 1990).

[100] The pH of a pharmaceutical composition or dosage form may also be adjusted to improve delivery of one or more active components. Similarly, the polarity of a solvent carrier, its ionic strength, or tonicity may be adjusted to improve the delivery. Compounds such as stearates may also be added to pharmaceutical compositions or dosage forms to advantageously alter the hydrophilicity or lipophilicity of one or more active components so as to improve the delivery. In this regard, stearates may serve as a lipid vehicle for the formulation, as an emulsifying agent or surfactant, and as a delivery-enhancing or uptake-enhancing agent. Different salts, hydrates or solvates of the active components may be used to further adjust the physical properties of the resulting composition.

[101]

### Advantageous Effects

[102] As described above, since the nuclease antibody according to one exemplary embodiment of the present invention has binding activity and degrading activity to the nucleic acid strands in cells at the same time, the nuclease antibody may be useful to show the cytotoxicity by damaging nucleic acid strands, that is, genetic information of cancer cells when the anti-nucleic acid antibody is overexpressed in the cancer cells, or flows into the cancer cell. Also, the nuclease antibody according to one exemplary embodiment of the present invention may be useful to be used for the composition for treating cancers capable of inducing the selective cell death in cancer cells than in normal cells since the anti-nucleic acid antibody very easily permeates into the cancer cells due to the excellent selectivity, compared to the normal cells.

[103]

### Brief Description of Drawings

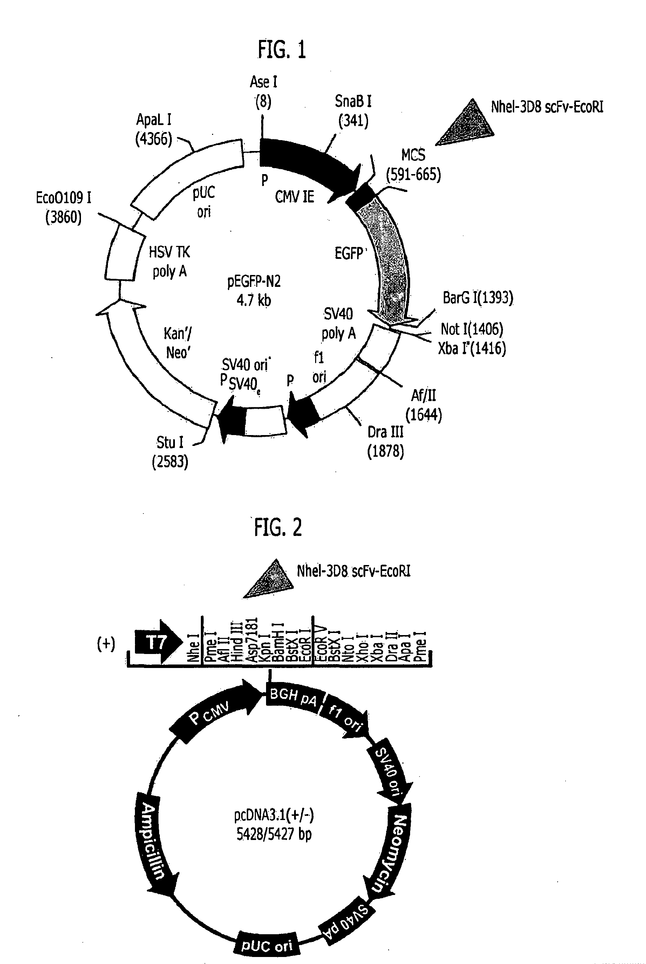

[104] FIG. 1 shows a cleavage map of an expression vector (pEGFP-N2) used to overexpress a 3D8 scFv protein of the present invention in cancer cells.

[105] FIG. 2 shows a cleavage map of an expression vector (pcDNA 3.1) used to overexpress a 3D8 scFv protein of the present invention in cancer cells.

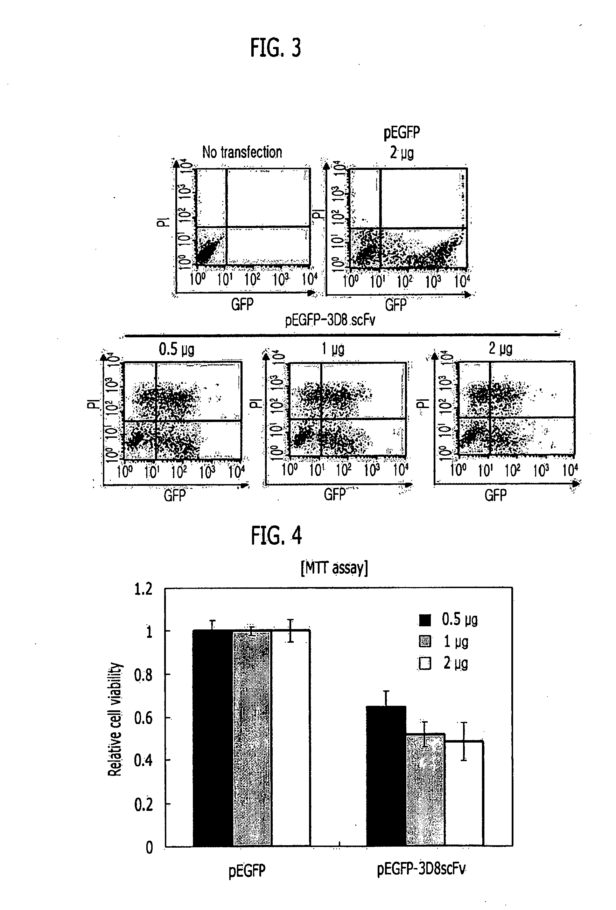

[106] FIG. 3 shows the measurement of the cytotoxicity using a flow cytometer after a 3D8 scFv protein is overexpressed from a 3D8 scFv protein expression vector (pEGFP-3D8 scFv) of the present invention in a human uterine cancer cell line (HeLa).

[107] FIG. 4 shows the measurement of the cytotoxicity using an MTT reagent after a 3D8 scFv protein is overexpressed from a 3D8 scFv protein expression vector (pEGFP-3D8 scFv) of the present invention in a HeLa cell line.

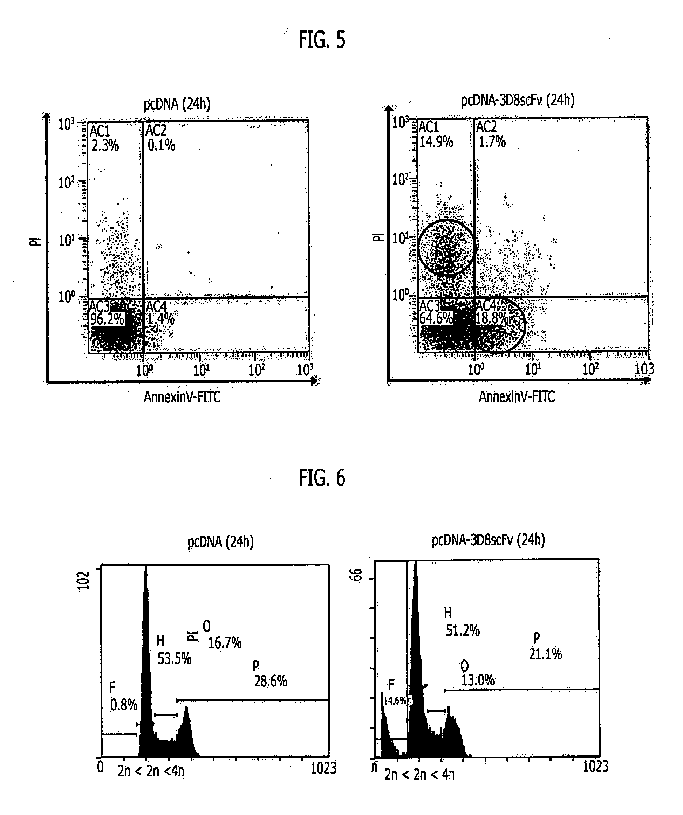

[108] FIG. 5 shows the measurement of a level of the cell death using a flow cytometer after a 3D8 scFv protein is overexpressed from a 3D8 scFv protein expression vector (pcDNA-3D8 scFv) of the present invention in a human uterine cancer cell line (HeLa).

[109] FIG. 6 shows that a DNA level in cells is reduced when a 3D8 scFv protein is overexpressed from a 3D8 scFv protein expression vector (pcDNA-3D8 scFv) of the present invention in a HeLa cell line, and genomic DNA is then stained with PI and measured in a flow cytometer.

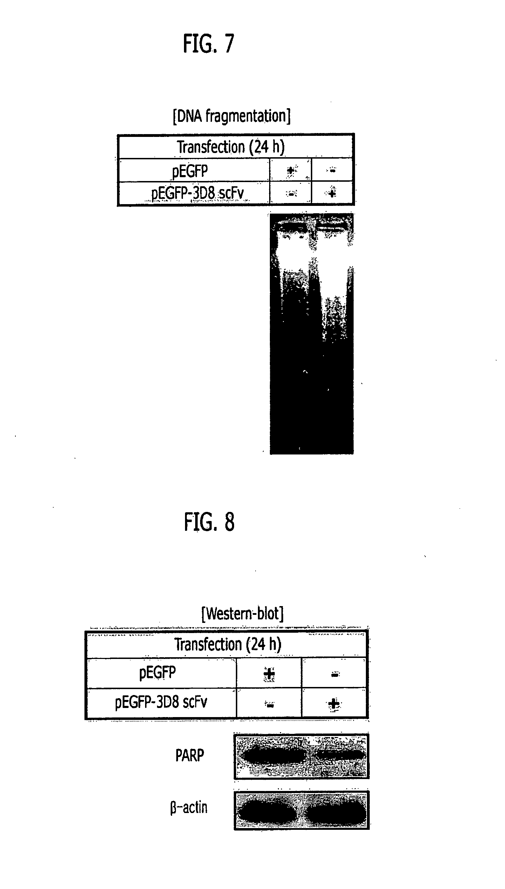

[110] FIG. 7 shows the electrophoretic results obtained by overexpressing 3D8 scFv protein in a HeLa cell line using a 3D8 scFv protein expression vector (pEGFP-3D8 scFv) the present invention and extracting DNA genome to determine fragmentation of genomic genes.

[111] FIG. 8 shows the western blotting detection of cleaved PARP protein from cell lysate after a 3D8 scFv protein is overexpressed from a 3D8 scFv protein expression vector (pEGFP-3D8 scFv) of the present invention in a HeLa cell line.

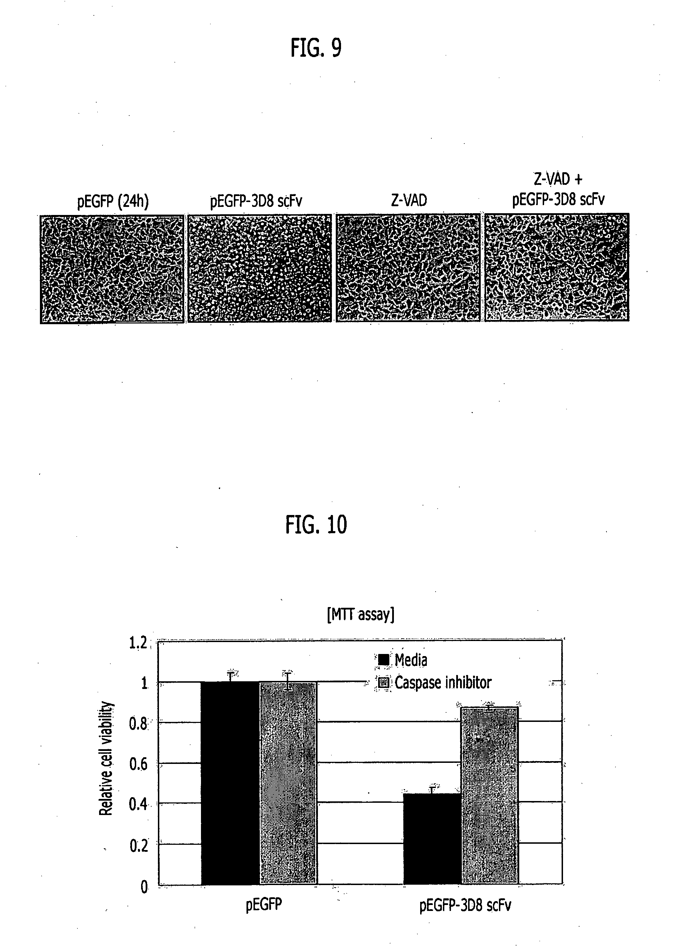

[112] FIG. 9 is the phase-contrast microscopic results showing that the cell viability is recovered when a HeLa cell line is pre-treated with a caspase inhibitor, z-VAD, and a 3D8 scFv protein is overexpressed from a 3D8 scFv expression vector (pEGFP-3D8 scFv) in the pre-treated HeLa cell line.

[113] FIG. 10 shows the measurement of the cell viability using an MTT reagent after a HeLa cell line is pre-treated with a caspase inhibitor, z-VAD, and a 3D8 scFv protein is then overexpressed from a 3D8 scFv protein expression vector in the pre-treated HeLa cell line.

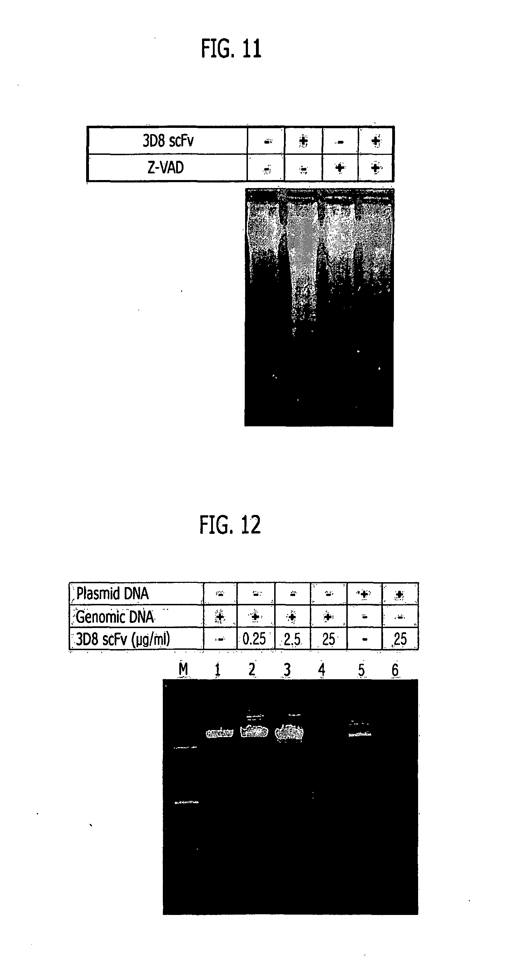

[114] FIG. 11 shows the electrophoretic results obtained by pre-treating a HeLa cell line with a caspase inhibitor, z-VAD, overexpressing 3D8 scFv protein in the pre-treated HeLa cell line using a 3D8 scFv protein expression vector (pEGFP-3D8 scFv) and extracting DNA genome to determine fragmentation of genomic genes.

[115] FIG. 12 is the electrophoretic results showing that the 3D8 scFv protein degrades the DNA genome extracted from the HeLa cell line and bacteria-derived plasmid DNA.

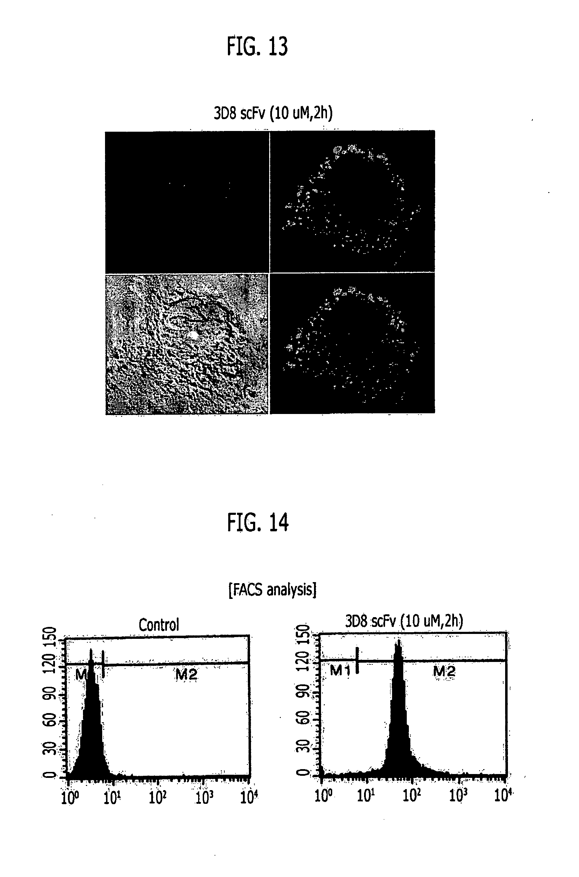

[116] FIG. 13 is the confocal fluorescent microscopic results showing that the 3D8 scFv protein permeates into a HeLa cells and is present in the cytoplasm when the HeLa cell line is treated with 3D8 scFv protein in a TOM medium and stained with an anti-3D8 scFv antibody.

[117] FIG. 14 shows the measurement of an uptake level of 3D8 scFv protein into a HeLa cell line using a flow cytometer when the HeLa cell line is treated with the 3D8 scFv protein in a TOM medium and stained with an anti-3D8 scFv antibody.

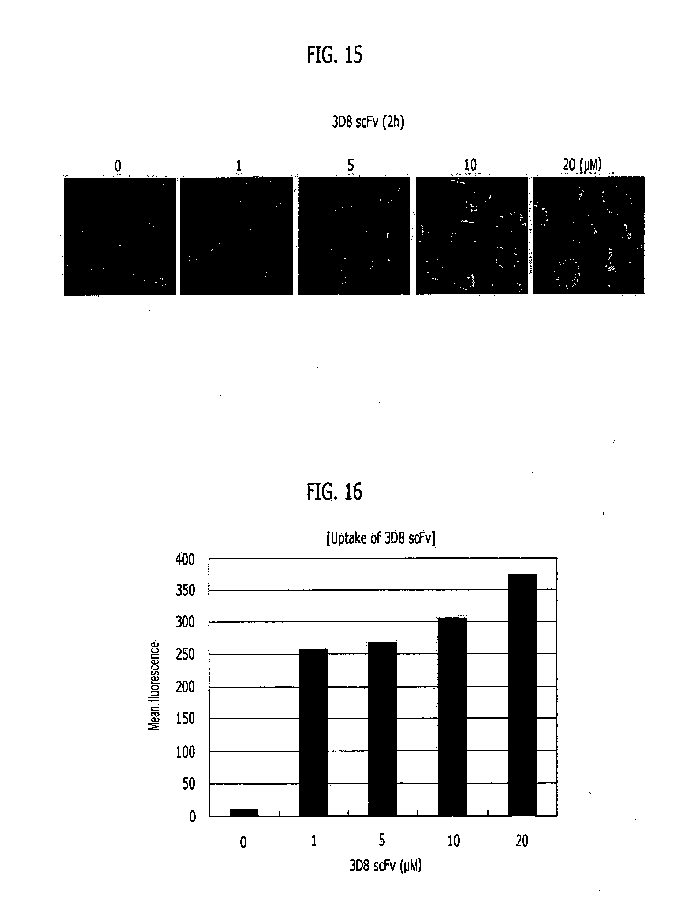

[118] FIG. 15 is the confocal fluorescent microscopic results showing an uptake level of 3D8 scFv protein into a HeLa cell line according to the concentration of the 3D8 scFv protein, as measured after the anti-3D8 scFv protein is stained with an anti-3D8 scFv antibody.

[119] FIG. 16 shows the measurement of an uptake level of 3D8 scFv protein into a HeLa cell line according to the concentration of the 3D8 scFv protein, as measured with a flow cytometer when the HeLa cell line is stained with an anti-3D8 scFv antibody.

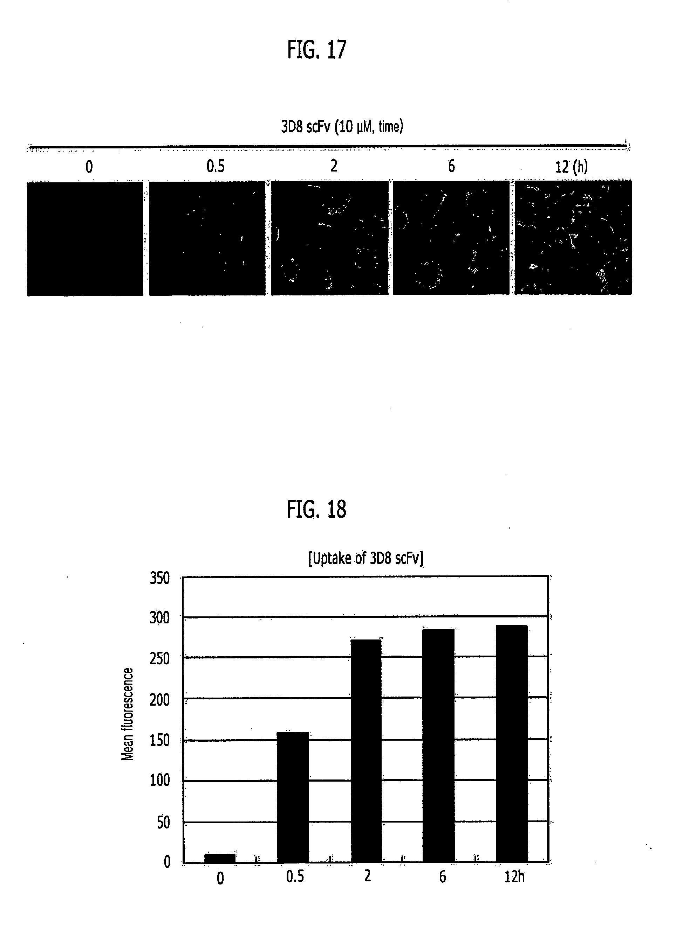

[120] FIG. 17 is the confocal fluorescent microscopic results showing an uptake level of 3D8 scFv protein into a HeLa cell line according to the culture time of the 3D8 scFv protein, as measured after the anti-3D8 scFv protein is stained with an anti-3D8 scFv antibody.

[121] FIG. 18 shows the measurement of an uptake level of 3D8 scFv protein into a HeLa cell line according to the culture time of the 3D8 scFv protein, as measured with a flow cytometer when the HeLa cell line is stained with an anti-3D8 scFv antibody.

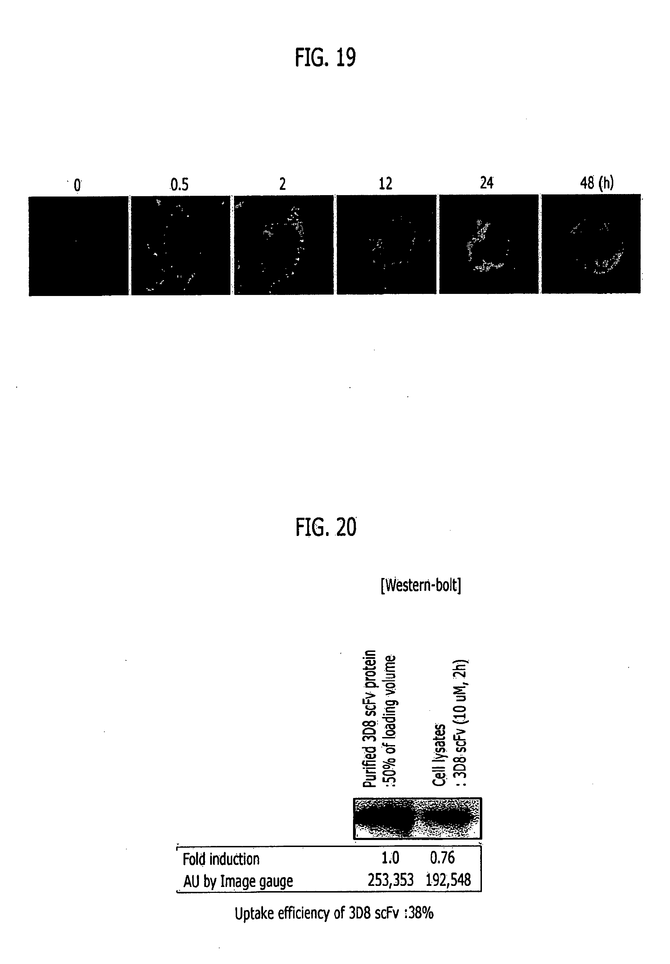

[122] FIG. 19 is the confocal fluorescent microscopic results showing the change in position of 3D8 scFv protein after the 3D8 scFv protein permeated into a HeLa cell line, as measured after the anti-3D8 scFv protein is stained with an anti-3D8 scFv antibody.

[123] FIG. 20 is the western blotting results showing an uptake efficiency of 3D8 scFv protein into a HeLa cell line.

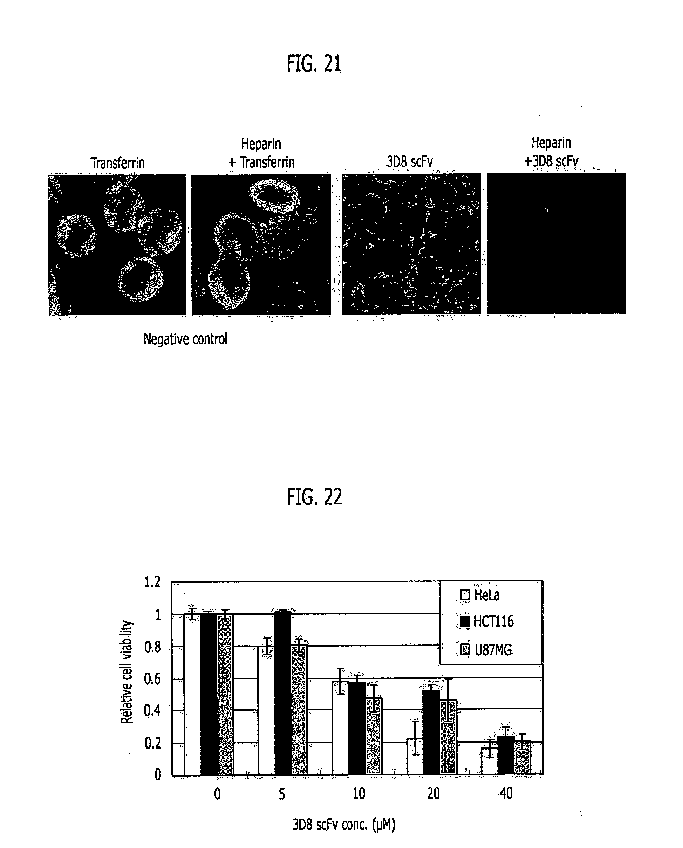

[124] FIG. 21 is the confocal fluorescent microscopic results showing that heparin sulfate proteoglycans (HSPGs) are associated with the interaction of 3D8 scFv protein and the cell membrane.

[125] FIG. 22 shows the measurement of the cytotoxicity using an MTT reagent after a HeLa cell line is treated with various concentrations of 3D8 scFv protein in a TOM medium.

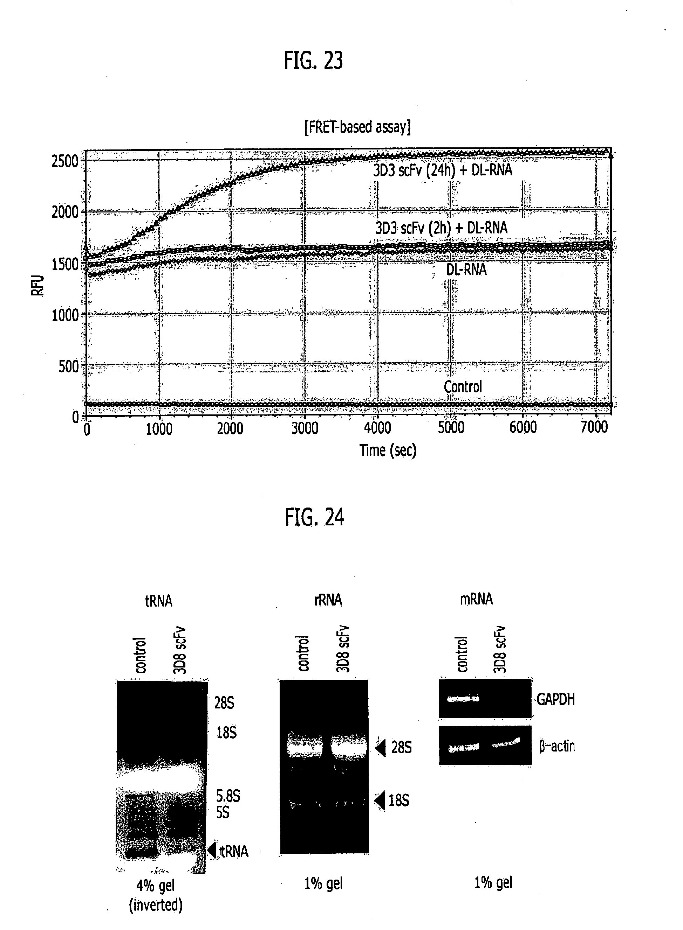

[126] FIG. 23 is the FRET (fluorescence resonance energy transfer) analysis results showing that synthestic RNA fragments are degraded after a HeLa cell line is treated with 3D8 scFv protein in a TOM medium.

[127] FIG. 24 is the electrophoretic results showing damage levels of tRNA, rRNA and mRNA, as measured when a HeLa cell line is treated with 3D8 scFv protein in a TOM medium and the total RNA is extracted from the HeLa cell line.

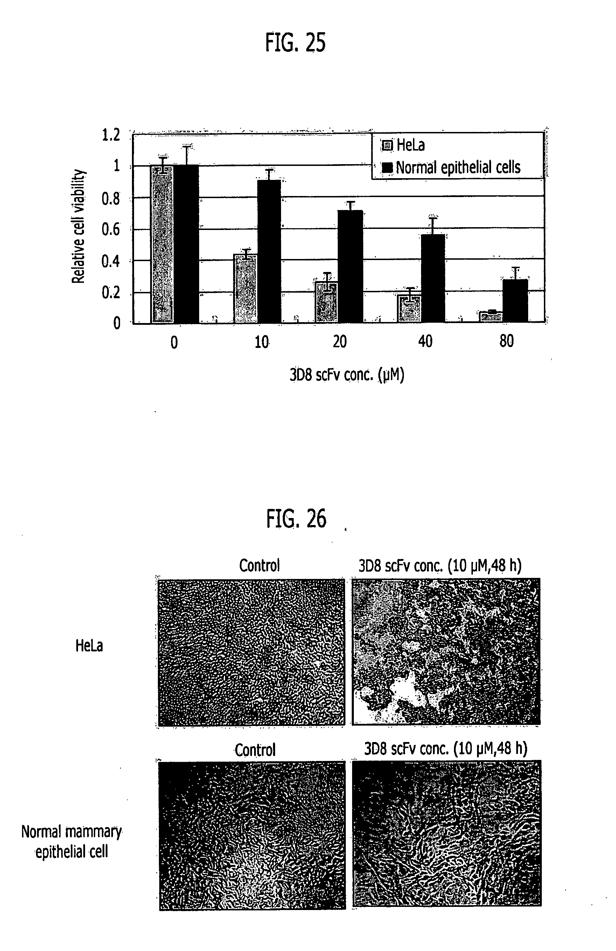

[128] FIG. 25 shows the comparison of the cytotoxicities using an MTT reagent, as measured after a HeLa cancer cell line and a normal mammary epithelial cell line are treated with 3D8 scFv protein.

[129] FIG. 26 is a phase-contrast microscopic photograph showing the cytotoxicities using an MTT reagent, as measured after a HeLa cancer cell line and a normal mammary epithelial cell line are treated with 3D8 scFv protein.

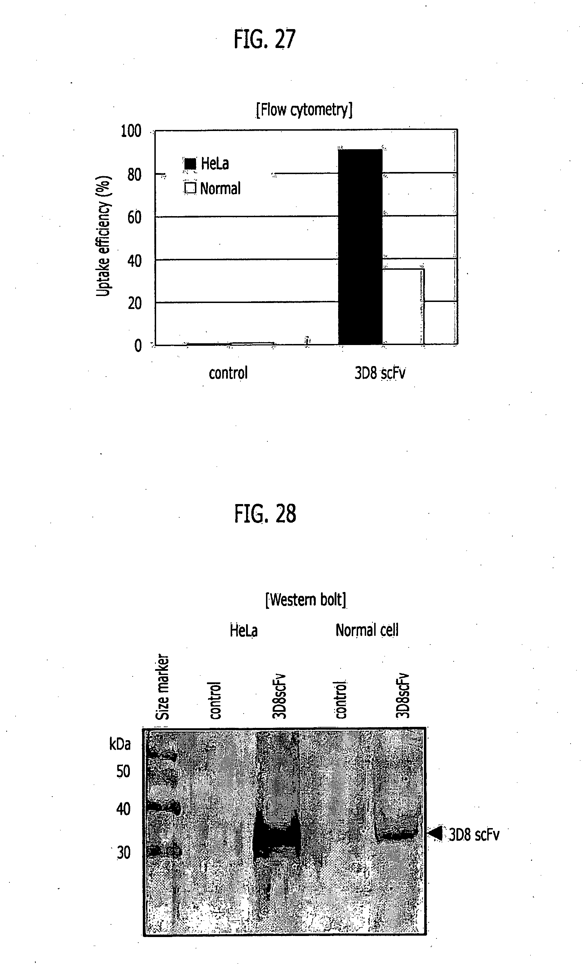

[130] FIG. 27 shows the comparison of uptake levels of 3D8 scFv protein into a HeLa cancer cell line and a normal mammary epithelial cell line, as measured after the two cell lines are treated with the 3D8 scFv protein.

[131] FIG. 28 shows the western blotting comparison of uptake levels of 3D8 scFv protein into a cancer cell line and a normal cell line, as measured after the two cell lines are treated with the 3D8 scFv protein.

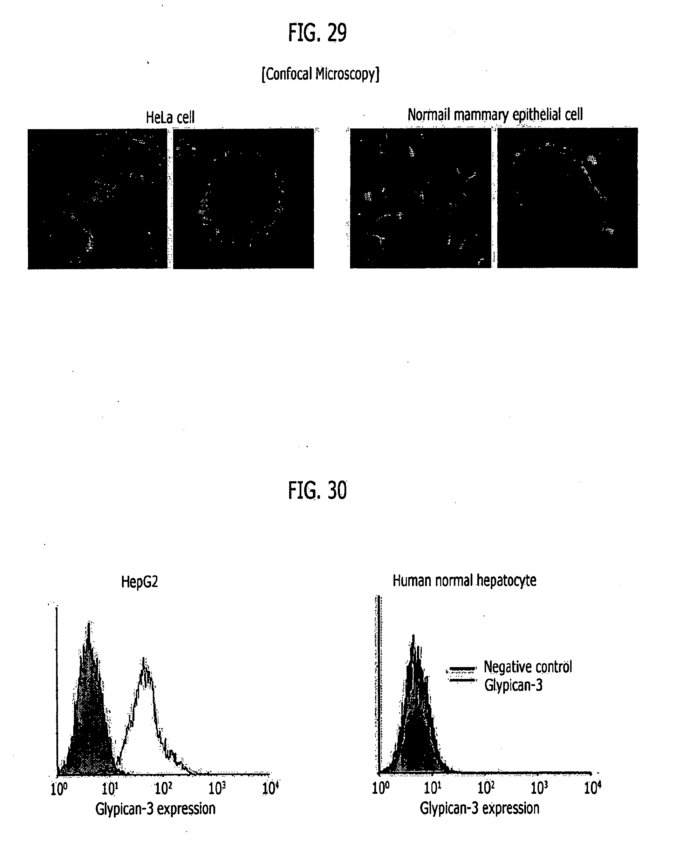

[132] FIG. 29 is the confocal fluorescent microscopic results showing uptake levels of 3D8 scFv protein into a cancer cell line and a normal cell line, as measured after the two cell lines are treated with the 3D8 scFv protein.

[133] FIG. 30 shows the comparison of expression levels of glypican-3 in a HepG2 liver cancer cell line and a human normal hepatocyte by using a flow cytometer.

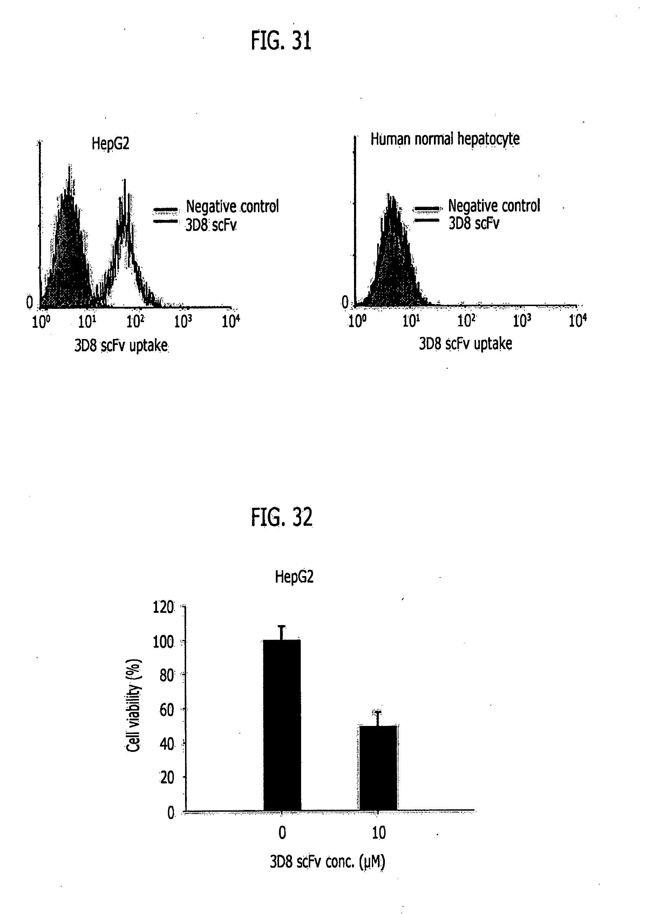

[134] FIG. 31 shows the comparison of uptake levels of 3D8 scFv protein into a HepG2 liver cancer cell line and a human normal hepatocyte, as measured with a flow cytometer after the two cell lines are treated with the 3D8 scFv protein.

[135] FIG. 32 shows the cytotoxicity using an MTT reagent, as measured after a HepG2 liver cancer cell line is treated with 3D8 scFv protein.

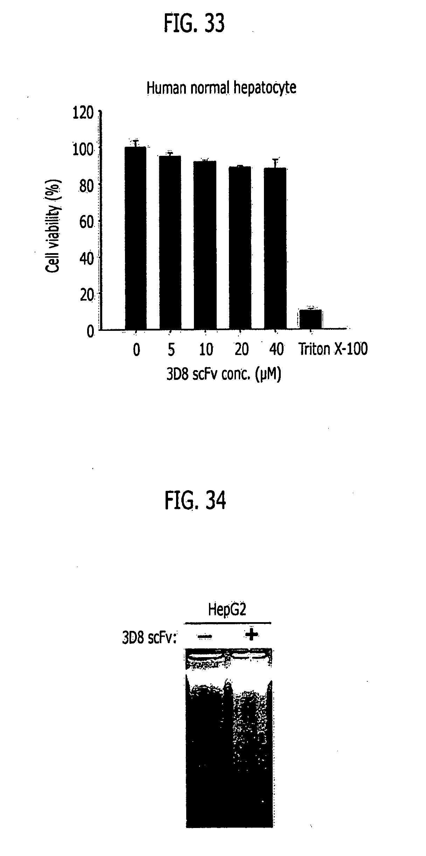

[136] FIG. 33 shows the electrophoretic results obtained by treating a HepG2 liver cancer cell line with 3D8 scFv protein, and extracting DNA genome to determine fragmentation of genomic genes.

[137] FIG. 34 shows the cytotoxicity using an MTT reagent, as measured after a human normal hepatocyte is treated with 3D8 scFv protein.

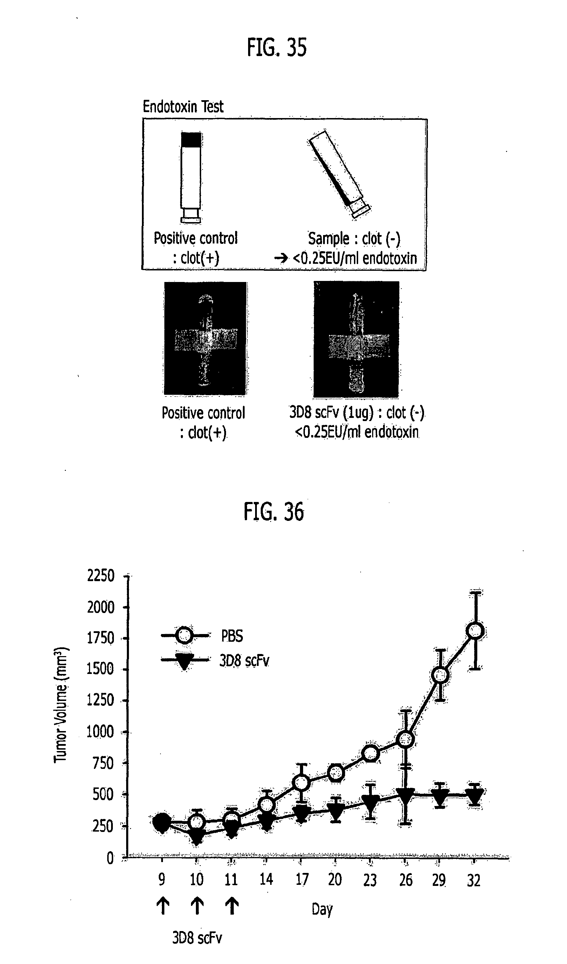

[138] FIG. 35 shows the gel clot assay results obtained by measuring a content of lipopolysaccharide (LPS) in 3D8 scFv protein purified from bacteria.

[139] FIG. 36 shows a tumor volume of cancer tissues, as measured when a HepG2 liver cancer cell line is transplanted and proliferated in a nude mouse and 3D8 scFv is introduced into the nude mouse.

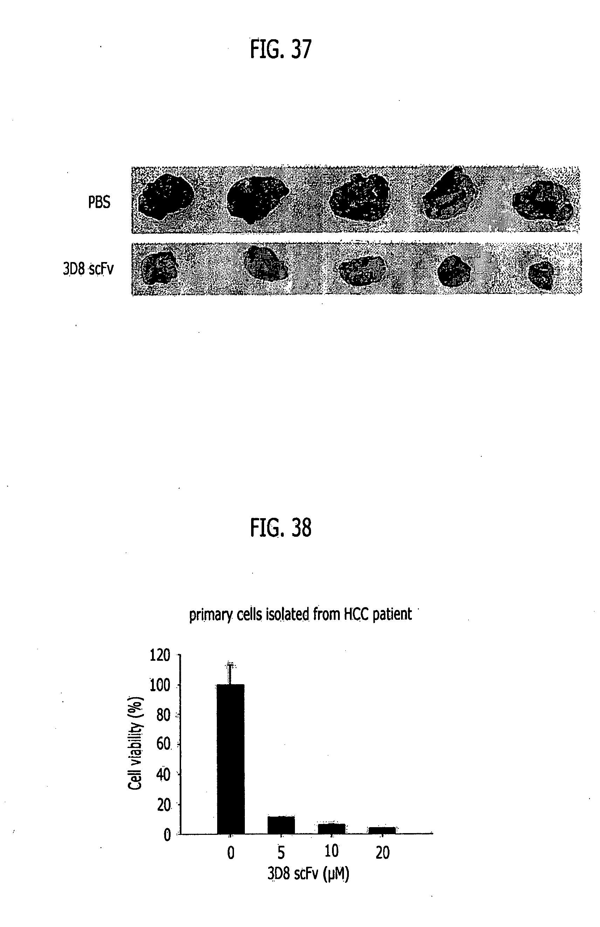

[140] FIG. 37 is a photograph showing cancer tissues taken from a nude mouse after a HepG2 liver cancer cell line is transplanted and proliferated in a nude mouse and 3D8 scFv is introduced into the nude mouse.

[141] FIG. 38 shows the cytotoxicity using an MTT reagent, as measured after a liver cancer cell isolated from a liver cancer patient is treated with 3D8 scFv protein.

[142]

### Best Mode for Carrying out the Invention

[143] Hereinafter, non-limiting exemplary embodiments of the present invention are described in more detail.

[144] Example 1: Construction, expression and purification **of 3D8 scFv protein expression vector**

[145] In order to purify scFv protein, a pIg20-3D8 scFv vector was transformed into E.coli BL21 DE3 (pLysE) cell (Novagen). 0.2 ml of a RNA extraction solution (RNAzol B, TEL-TEST) was added to approximately 1x10⁶ 3D8 hybridoma cells (deposited in the Korean Cell Line Research Foundation (KCLRF): KCLRF-BP-00146), and the 3D8 hybridoma cells was lysed and homogenated. 0.02 ml of chloroform was added to the cell homogenate, and mixed thoroughly for 15 seconds, and the resulting cell mixture was kept on ice for 5 minutes. The cell mixture was centrifuged to isolate a supernatant, and 0.25 ml of ethanol was added to the supernatant. Then, the supernatant was kept at 4°C for 15 minutes, and centrifuged (12,000 g, 4°C) for 15 minutes. A RNA precipitate was washed with 70% ethanol, dried and dissolved in distilled water. cDNA was synthesized from the extracted RNA using a RT-PreMix kit (Bioneer). In this case, oligo-(dT) oligonucleotides were used as the primers. Then, VH and VL genes were amplified by PCR using the obtained cDNA as a template. A pair of primers (5'-ATGGGATGGAGCTRTATCATSYTCTT-3' (SEQ ID NO: 7) and 5'-TGGATAGACAGATGGGGGTGTCGTTTTGGC-3'(SEQ ID NO: 8)) were used 40 amply the VH gene, and a pair of primers (5'- ATGAAGTTGCCTGTTAGGCT-GTTGTGTCTC-3' SEQ ID NO: 9) and 5'- GGATGGTGGGAAGATGGATAC-3' (SEQ ID NO: 10)) were used to amply the VL gene. The amplified VH gene (approximately 360 bp) and VL gene (approximately 340 bp) were sequentially subcloned into a pIg20 vector (kindly provided by Professor Stollar BD of Turfs University, USA) to establish a pIg20-3D8 scFv expression vector. In this case, the primers used to clone the VH gene were designed to include an XmaI/XbaI restriction enzyme recognition site, and the primers used to clone the VL gene were designed to include a BglII/NcoI restriction enzyme recognition site. The transformed BL21 DE3 (pLysE) strain was incubated in a LB broth supplemented with ampicillin (100 *µ*g/ml) and chloramphenicol (20 *µ*g/ml) until the absorbance A600 value reached 0.8. Isopropyl-β-D-thiogalactopyranoside (IPTG) was added in a concentration of 0.5 mM, and the BL21 DE3 (pLysE) strain was further incubated at a room temperature for 4 hours, and then centrifuged at a rotary speed of 10,000 xg at 4°C for 10 minutes to obtain a supernatant. Then, the supernatant was filtered through a 0.45 *µ*m membrane. The resulting filtrate was passed through an IgG-Sepharose column (Amersham pharmacia Biotech) (1 ml/min), washed with a phosphate buffered solution (PBS, pH 7.4) and ammonium acetate (pH 5.0), and then eluted with 0.1 M acetic acid (pH 3.4) to obtain a desired protein. The eluted protein was dialyzed in a PBS solution, and analyzed for protein purity under a reduction condition using SDS-PAGE. Approximately 20 *µ*g of the protein was electrophoresized on SDS-PAGE, and stained with coomassie blue. In this case, it was confirmed that the scFv antibody is purified with a purity of 95% or more.

[146]

[147] **Example 2: Construction of vector to express 3D8 scFv in animal cell**

[148] In order to express a recombinant single chain variable fragment (3D8 scFv) in animal cells, two vectors, that is, pEGFP-N2 (Clontech) and pcDNA 3.1 (+) vector (Clontech) were used. The pEGFP-N2 vector was designed to express the 3D8 scFv along with an enhanced green fluorescence protein (EGFP) at the C-terminus of the 3D8 scFv, and the pcDNA 3.1 (+) vector was designed to express only the 3D8 scFv. A PCR using the pIg20-3D8 scFv vector constructed in Example 1 as the template was performed to amplify the 3D8 scFv gene, and the amplified 3D8 scFv gene was electrophoresized on 1% agarose gel. Then, the scFv gene (approximately 750 bp) was extracted from the agarose gel, was cloned between NheI and EcoRI restriction enzyme recognition sites of the pEGFP-N2 vector, and also cloned between NheI and EcoRI restriction enzyme recognition sites of the pcDNA3.1 vector (FIGS. 1 and 2).

[149]

[150] **Example 3: Forms of VH and VL proteins and their associatin**

[151] A HPLC **system** was used to perform the size exclusion chromatography (SEC) analysis on the protein of the purified VH, VL, scFv and Fv (a non-covalent VH and VL chain conjugate). 0.02 ml of 5 to 20µM protein was injected into a TSK G3000SW XL column (TosoHaas, Japan), and a phosphate buffered solution (50 mM sodium phosphate/150 mM NaCl, pH7.4) flowed at a rate of 0.7 ml/min. In this case, the chromatogram was obtained by measuring absorbance values at 280 nm.

[152] It was observed that the VH and VL chains were present in the form of monomer in the phosphate buffered solution, but they were not present in the form of multimer.

[153] Meanwhile, it was confirmed that Fv fragments that are formed by the spontaneous association of the VH and VL chains are observed between the VH and VL domains. The quantitative association between the VH and VL chains was analyzed using surface plasmon resonance (SPR) (Biacore 2000 SPR biosensor, Pharmacia, Sweden).

[154] The VL protein (0.5 ∼ 1.0 mg/ml) was immobilized onto a carboxymethylated dextran surface of a CM5 sensor chip (Amersham pharmacia Biotech), and the VH protein (12.5, 25, 50, 100 and 200 nM) run on the CM5 sensor chip in a phosphate buffered solution, thus to analyze the sensorgram. As a result, it was confirmed that the VH protein binds to the VL protein with affinity of K_{D} 14 nM.

[155]

[156] **Experimental example 1: Evaluation of cytotoxic effect by expression of 3D8 scFv in cell using pEGFP-3D8 scFv vector**

[157] <1-1> Cell culture

[158] A human cervix cancer cell line, HeLa, was cultured in a DMEM medium (supplemented with 10% fetal bovine serum (FBS), 100 units/ml penicillin and 100 ug/ ml streptomycin) in a 5% CO₂ incubator, and used for the cytotoxicity analysis.

[159]

[160] <1-2> Injection of nucleic acids into cells

[161] In order to overexpress the 3D8 scFv in a HeLacell line using a simple transfection method, a HeLa cell line was cultured in a 6 well plate 24 hours before the experiment (2x10⁵ cell /well), and washed twice with PBS right before the transformation. Then, the used medium was exchanged with transfection optimized medium (TOM). 10 *µ*ℓ of lipofectamine 2000 solution (Invitrogen) and 1 *µ*g of the pEGFP-N2 or pEGFP-3D8scFvvector (FIG. 1) were mixed thoroughly and kept at a room temperature for 15 minutes. The resulting mixture was added to each HeLa cell line cultured in the 6-well plate. After the 6 hour treatment, the used medium was exchanged with a DMEM medium supplemented with 10% FBS, and incubated for 24 hours, which was used later to confirm an expression level and cytotoxic effect of the 3D8 scFv.

[162]

[163] <1-3> Analysis of cytotoxicity by expression of 3D8 scFv in cell using Flow cytometry

[164] In order to determine the cytotoxicity of a nuclease antibody to cancer cells when the nuclease antibody was expressed in the cancer cells, the 3D8 scFv was expressed in a human cervix cancer cell line HeLa (Preparative example 2 and FIG. 3). 0.5, 1, and 2 ug of a pEGFP-N2 vector (negative control) and a pEGFP-3D8 scFv vector (FIG. 1) were introduced into cells, respectively, using Lipofectamine 2000 (Invitrogen, Carlsbad, CA), and incubated at 37°C for 24 hours in a CO₂ incubator. Then, an expression level of the 3D8 scFv and the cell death were analyzed using flow cytometry. That is, the cultured cells were washed twice with PBS, and suspended in 200 *µℓ* PBS. Then, 1 ul of propidium iodide (PI, 5 *µ*g/ml) was added to the cell suspension, and reacted at a room temperature for 10 minutes. Finally, the resulting cell suspension was analyzed using flow cytometry (FIG. 3). Also, the cytotoxicity of the nuclease antibody was re-analyzed using MTT assay (FIG. 4). That is, the cultured cells were transfected with the vector in a 96-well plate. After the 24 hour transfection, an MTT solution was added to the culture solution, and reacted at 37°C for 4 hours in a CO₂ incubator. Then, the culture solution was removed, and the cultured cells were suspended in DMSO to measure OD values at 595 nm (FIG. 4). As a result, it was confirmed that the 3D8 scFv protein was sufficiently expressed in the HeLa cell line, and that the higher an expression rate of the 3D8 scFv protein is, the lower the cell viability is.

[165]

[166] **Experimental example 2: Evaluation of cytotoxic effect by expression of 3D8 scFv in cell using pcDNA-3D8 scFv vector**

[167] The cytotoxicity by the expression of 3D8 scFv was re-confirmed with the expression of pure scFv to which EGFP is not fused. A HeLa cell line was cultured for 24 hours in a 6-well plate, and simply transfected with a pcDNA (negative control) and a pcDNA-3D8 scFv vector using Lipofectamine2000, and then incubated for a predetermined time period. Then, the cultured cells were washed twice with PBS. Then, a fluorescent FITC Annexin V (2.5 mg/ml) and propidium iodide (5 *µ*g/ml) (BD Pharmingen, San Diego, CA) were added to the cultured cells, and reacted at room temperature for 15 minutes. Then, a staining level of the cells was measured using flow cytometer. This is based on the fact that cells expressing 3D8 scFv were stained with PI that binds to DNA in the dying cells, and the Annexin V is detected when the cells are being apoptosized. As a result, it was revealed that the cell death was proportional to the higher expression rate of the 3D8 scFv in the cancer cells expressing 3D8 scFv, and that the cell death is mediated by apoptosis (FIG. 5). As a result, these results indicate that the 3D8 scFv induces the cytotoxicity when it is expressed in the cancer cells. Also, PI staining was carried out to determine how to trigger the cell death through the analysis of the change in amount of DNA in cells (FIG. 6). A HeLa cell line was cultured in a 6-well plate for 24 hours and the 3D8 scFv was overexpressed using a Lipofectamine2000 transfection method. Then, the HeLa cell line was incubated for 24 hours, washed twice with PBS, and immobilized with 70% ethanol. Then, the immobilized cells were stained with propidium iodide (5 *µ*g/ml) to analyze a cell cycle of the cells using a flow cytometer. Dying cells have less than 2n DNA, which is called sub G1. From these experimental results, it was confirmed that, when the 3D8 scFv is expressed, the cells with less than 2n DNA are increasingly observed, which indicates that the cell death is induced by the expression of the 3D8 scFv (FIG. 6).

[168]

[169] **Experimental example 3: Analysis of cell death pattern bv expression of 3D8 scFv in HeLa cell line**

[170] In order to determine whether a pattern of the cell death caused by the expression of 3D8 scFv in a HeLa cell line is induced by the apoptosis, DNA fragmentation and detection of cleaved PARP molecules (cleaved form) were performed (FIG. 7). 1 *µ*g of the pEGFP-3D8 scFv vector (FIG. 1) was injected into a HeLa cell line using Lipofectamine 2000, and the HeLa cell line was incubated at 37°C for 24 hours in a CO₂ incubator. In order to determine DNA cleavage, genomic DNA was extracted from the HeLa cell line (approximately 2 x 10⁵/ml cells) (using a genomic DNA extraction kit (G-DEX™IIc Invitrogen, Seoul, Korea)), and electrophoresized on 2% agarose gel supplemented with EtBr (FIG. 7). In order to detect cleaved PARP molecules, the resulting cell lysate was electrophoresized on SDS-PAGE gel, and then subjected to western blotting. After the 24-hour transfection, the cultured cells were washed twice with PBS, sufficiently dissolved in a cell lysis buffer (5 mM/L EDTA; 300mM/L NaCl; 0.1 % NP-40; 0.5 mM/L NaF; 0.5 mM/L Na₃VO₄;0.5 mM/L phenylmethyl-sulfonyl fluoride; and 10 *µ*g/mL of each of aprotinin, pepstatin and leupeptin; Sigma, St. Louis,MO), and then centrifuged at a rotary speed of 5,000 x g for 30 minutes in a centrifuge to obtain a supernatant. The obtained supernatant electrophoresized on 10% SDS-PAGE gel, and then transferred to a membrane. In this case, a rabbit anti-PARP antibody (Santa Cruz Biotech) was used as a primary antibody, and anti-rabbit IgG-HRP (Sigma) was used as a secondary antibody. Then, a chemiluminescencedetection system (Amersham Pharmacia Biotech, Uppsala, Sweden) was used to visualize signals of the PARP molecule, and an X-ray film was exposed, and then developed (FIG. 8). As a result, it was revealed that the genomic DNA was fragmented with constant length (FIG. 7), and a pattern of the cell death caused by expression of a catalytic antibody is induced by the apoptosis by detecting a cleaved form of PARP proteins (FIG. 8). Also, when cells were treated with an apoptosis inhibitor, z-VAD (10uM), it was observed that the cell viability is recovered (FIGS. 9 and 10), and the fragmentation of the genomic DNA is inhibited (FIG. 11), which re-indicates that the cells are dying by the apoptosis mechanism (FIGS. 9 to 11).

[171]

[172] **Experimental example 4: Evaluation of DNA-degrading activity of 3D8 scFy**

[173] 300 ng of genomic DNA extracted from the HeLa cell line or 300 ng of plasmid DNA was reacted with the 3D8 scFv protein, which was purified from various concentrations (0.25, 2, 5, and 25 ug/ml) of bacteria, at 37°C for 1 hour in a TBS solution supplemented with Mg²⁺ ions. Then, the resulting reaction mixture was electrophoresized on 1% agarose gel containing EtBr. As a result, it was confirmed that the 3D8 scFv protein shows the degrading activity against the genomic DNA as well as the plasmid in a concentration-dependent manner (FIG. 12). This indicates that, when the 3D8 scFv is expressed in the cancer cells, the 3D8 scFv protein may reduce the DNA damage in the cancer cell line.

[174]

[175] **Experimental example 5: Determination of whether 3D8 scFv by itself permeates into cells**

[176] By using a confocal microscopy and a flow cytometer, it was confirmed that, when cells were treated with 3D8 scFv, the 3D8 scFv by itself permeated into the cells (FIGS. 13 to 14). For the confocal microscopy, a HeLa cell line was put into each well of a 24-well plate on which a sterile cover glass was put, and incubated for 24 hours [2x10⁵ cell /well]. After the 24-hour cell culture, the cultured cells were washed twice with PBS. Then, the used medium was exchanged with a fresh TOM medium, and kept for 30 minutes. Then, each well was treated with 10 mM of the TOM medium, and incubated for a certain time period. After the cell culture, the immunofluorescence staining was performed to observe 3D8 scFv protein permeating into the cells using a microscope. That is, after the used medium was removed from each well, the cells were washed once with PBS buffer supplemented with 2% FBS. Subsequently, 400 *µℓ* of a fixation/permeabilization solution (Becton Dickinson) was added to each well, and kept at 4°C for 20 minutes. After the resulting cells were washed twice with PBS buffer supplemented with 2% FBS, 500 *µ*ℓ of rabbit serum containing 3D8 scFv antibody (diluted 1:500) was also added to each well, and the washed cells were reacted with the 3D8 scFv antibody at a room temperature for 60 minutes, and then washed three times with PBS buffer supplemented with 2% FBS. Then, 500 ul of an anti-rabbit antibody (diluted 1:500 with a 2% FBS-containing PBS buffer) to which a fluorescent TRIRC is covalently bonded was added to each well, the washed cells were reacted with the anti-rabbit antibody at a room temperature for 30 minutes, washed three times with the 2% FBS-containing PBS buffer, and immobilized with 4% PFA at a room temperature for 20 minutes. 3 *µ*ℓ of a fixing solution (Invitrogen) containing a reagent, DAPI (4'-6-Diamidino-2-phenylindole), that stains the nucleus blue was added to the prepared slide glass, and the slide glass was covered with a cover glass to which the cells were attached, and the outskirts of the glasses were sealed with a fixing solution to observe the 3D8 scFv protein (red fluorescence) in the cells using a confocal microscope. As a result, it was confirmed that, when the HeLacell line was treated with the 10 µM 3D8 scFv protein for 2 hours, the 3D8 scFv protein readily permeates into the cytoplasm of the HeLa cell line (FIG. 13).

[177] In order to quantify the 3D8 scFv-permeated cells, the flow cytometry were performed (FIG. 14). A HeLa cell line was added to each well of a 6-well plate and cultured for 24 hours [3x10⁶ cell /well (well)]. After the 24-hour cell culture, the cultured cells were washed twice with PBS. Then, the used medium was exchanged with a fresh TOM medium, and incubated for 30 minutes. Then, each well was treated with 10 mM of the TOM medium, and incubated for a certain time period. After the used medium was removed from each well, the cells were then treated with TE (Trypsine/EDTA) to separate the cells from each well, and washed twice with PBS buffer. Subsequently, 400 *µ*ℓ of a fixation/permeabilization solution (Becton Dickinson) was added to each well and kept at 4°C for 20 minutes. After the resulting cells were washed once with PBS buffer, 500 *µ*ℓ of rabbit serum containing 3D8 seFv antibody (diluted 1:500) was added to each well, and the washed cells were reacted with the 3D8 scFv antibody for 60 minutes on ice, and then washed three times with PBS buffer. Then, 500 *µ*ℓ of a solution of anti-rabbit antibody (diluted 1:500 with a 2% FBS-containing PBS buffer) to which a fluorescent TRIRC is covalently bonded was added to each well, the washed cells were reacted on ice with the anti-rabbit antibody for 30 minutes, washed three times with PBS buffer, and immobilized with 4% PFA at a room temperature for 20 minutes. After the immobilization of the cells, a ratio of the 3D8 scFv protein permeating into the cells was analyzed using a flow cytometer. As a result, it was confirmed that the 3D8 scFv protein permeates into almost all the cells (FIG. 14).

[178]

[179] **Experimental example 6: Uptake level of 3D8 scFv according to concentration and culture time of 3D8 scFv**

[180] In order to determine an uptake level of 3D8 scFv according to the concentration and culture time of the 3D8 scFv, the confocal microscopy and immunofluorescence flow cytometry were performed (FIGS. 15 to 18). These general experimental procedures were performed in the same manner as in Experimental example 5. In order to determine an uptake level of 3D8 scFv according to the concentration of the 3D8 scFv, a HeLa cell line was treated with an increasing concentration (1, 5, 10, and 20 µM) of 3D8 scFv protein. After the 2-hour treatment, the HeLa cell line was observed (FIGS. 15 and 16). Also, in order to determine an uptake level of 3D8 scFv according to the culture time of the 3D8 scFv, a HeLa cell line was treated with 10 µM 3D8 scFv, incubated for 0.5, 2, 6 and 12 hours, and then observed (FIGS. 17 and 18). As a result, it was confirmed that the uptake level of the 3D8 scFv protein is in proportion to the concentration and culture time of the 3D8 scFv protein.

[181]

[182] **Experimental example 7: Distribution pattern of 3D8 scFv in cells according to culture time after uptake of 3D8 scFv**

[183] In order to determine a distribution pattern of 3D8 scFv according to the culture time after the uptake of the 3D8 scFv, the confocal microscopy was performed (FIG. 19). These general experimental procedures were performed in the same manner as in Experimental example 5. A HeLa cell line was treated with 3D8 scFv protein (10 uM) for 30 minutes, and observed at time points of 0.5, 2, 12, 24, 48 hours using a confocal microscope. As a result, it was confirmed that the 3D8 scFv protein is uniformly distributed in the cytoplasm and is not present in the nucleus as the time goes on (FIG. 19).

[184]

[185] **Experimental example 8: Uptake efficiency of 3D8 scFv**

[186] A HeLa cell line was culture in a 6-well plate for 24 hours, incubated in a TOM medium at 37°C for 30 minutes in a CO₂ incubator, and treated with 3D8 scFv protein (10 µM/2 hours). Then, the total proteins were extracted from the HeLa cells, and subjected to western blotting, thus to analyze an uptake efficiency of the 3D8 scFv protein into cells (FIG. 20). The western blotting was performed in the same manner as in Experimental example 3. The uptake efficiency of the 3D8 scFv protein was obtained by comparing an image with a band intensity of the purified 3D8 scFv protein and calculating the comparison data into a numeral value (%). As a result, it was confirmed that, when the 3D8 scFv was treacted in a concentration of 10 µM for 2 hours, approximately 38% of the 3D8 scFv protein permeates into the HeLa cells

[187]

[188] **Experimental example 9: Confirmation of molecule that interacts with cell membrane in uptake of 3D8 scFv protein**

[189] In order to determine whether 3D8 scFv protein interacts with a component, heparan sulfate proteoglycan (HSPG), of the cell membrane in the uptake of the 3D8 scFv protein, the confocal microscopy was performed (FIG. 21). These general experimental procedures were performed in the same manner as in Experimental example 5. A HeLa cell line was kept in a TOM medium for 30 minutes (at 37°C in a CO₂ incubator), pre-treated for 30 minutes with water-soluble heparin (100 U/ml) that is a competitive inhibitor of HSPGs, and then treated with 3D8 scFv protein (10 uM). The resulting HeLa cells were incubated at 37°C for 2 hours in a CO₂ incubator, and an uptake level of the 3D8 scFv protein permeating into the HeLa cells was analyzed using a confocal microscope. As a result, it was confirmed that, unlike transferrin used as the negative control, the uptake of the 3D8 scFv into the HeLa cells is completely inhibited by the treatment with water-soluble heparin. This indicates that the 3D8 scFv interacts with HSPGs of the cell membrane in the uptake into the HeLa cells (FIG. 21).

[190]

[191] **Experimental example 10: Cytotoxicity of 3D8 scFv in other cancer cell lines except for HeLa cell**

[192] The cell death was induced when HeLa cells were treated with 3D8 scFv. In this case, in order to calculate an EC50 value and also to determine the cytotoxicity of 3D8 scFv to other cancer cell lines, HCT116 (human colon cancer cell line) and U87MG (human glioma cell line), each cancer cell line was treated with various concentrations of 3D8 scFv protein, and subjected to an MTT assay after the 48-hour treatment (FIG. 22). As a result, it was confirmed that the cytotoxicity of the 3D8 scFv concentration in the HeLa, HCT116 and U87MG cells is increased in proportion to the concentration of the 3D8 scFv protein (FIG. 22).

[193]

[194] **Experimental example 11 : Determination of ribonucleic acid (RNA) damage by uptake of 3D8 scFv into cancer cell**

[195] In order to determine whether the RNA-degrading activity is maintained in cells and to analyze the RNA substrate specificity when the 3D8 scFv protein having a nuclease activity permeates into the cells, the fluorescence resonance energy transfer (FRET) assay and agarose gel electrophoresis were performed (FIGS. 23 and 24). For the FRET assay (FIG. 23), a HeLa cell line was kept in a TOM medium for 30 minutes (at 37°C in a CO₂ incubator), and incubated with 3D8 scFv protein (10 µM) for 2 hours or 24 hours. Then, a double-labeled, 24-base pair fragment of synthetic RNA having a fluorescent material FAM at the 5'-terminus and a black-hole quencher at the 3'-terminus was transfected into cells using Lipofectamine 2000, and the fluorescence intensity was measured in real time (0 - 2 hours) using a fluorescence detector to determine whether the 3D8 scFv protein degrades RNA substrate in the cells. As a result, it was confirmed that transfected RNA molecules are degraded in the cells 24 hours after the uptake of the 3D8 scFv into the cells, unlike the negative control where the 3D8 scFv does not permeated in the cells (FIG. 23). Meanwhile, for the agarose gel electrophoresis (FIG. 24), a HeLa cell line was kept in a TOM medium for 30 minutes (at 37°C in a CO₂ incubator), and incubated with 3D8 scFv protein (10 µM) for 2 hours, and then RNA was extracted from the HeLa cells. The extracted RNA was electrophoresized one 4% or 1% agarose gel supplemented with EtBr, or cDNA-was synthesized from the extracted RNA, and amplified in PCR method using primers specific to GAPDH or β-actin gene, thus to determine an expression level of the GAPDH and β-actin mRNA in the HeLa cells. As a result, the damages of tRNA and mRNA were clearly observed, compared to the rRNA accumulated by ribosomal proteins (FiG. 24). This indicates that the 3D8 scFv protein permeating into the cancer cells may be toxic to cancer cells since the 3D8 scFv protein induces the damage of tRNA or mRNA due to the abnormal mechanism of the cancer cells. [196]

[197] **Experimental example 12: Comparison of toxicities of 3D8 scFv protein in normal cell line and cancer cell line**

[198] In order to determine a level of toxicity of 3D8 scFv protein in a cancer cell line and a normal cell line, a HeLa cell line was used as the cancer cell line, and a human mammary epithelial cell line (Cambrex) was used as the normal cell line (FIGS. 25 and 26). The normal mammary epithelial cell line and the HeLa cancer cell line were treated with 3D8 scFv protein, and incubated at 37°C for 48 hours in a CO₂ incubator. After an MTT solution was added to each of the incubated cell lines, the incubated cell lines were reacted at 37°C for 4 hours in a CO₂ incubator. Subsequently, the culture solution was removed, and each cell line was suspended in DMSO, and its OD value was measured at 595 nm (FIG. 25). Here, shapes of the cells were observed using a phase-contrast microscope (FIG. 26). As a result, it was confirmed that the 3D8 scFv protein shows a stronger toxicity to the cancer cell line than to the normal cell line.

[199]

[200] **Experimental example 13: Comparison of uptake levels of 3D8 scFv into cells in normal cell line and a cancer cell line**

[201] In order to determine an uptake level of 3D8 scFv protein into cells in a cancer cell line and a normal cell line, a HeLa cell line was used as the cancer cell line, and a human mammary epithelial cell line (Cambrex) was used as the normal cell line. The normal cell line and the HeLa cancer cell line were treated with 3D8 scFv protein, incubated for 24 hours, and then analyzed (FIGS. 27 to 29). The uptake levels of the 3D8 scFv protein into cells were analyzed using immunofluorescence flow cytometry (FIG. 27), western blotting (FIG. 28), and confocal fluorescent microscopy (FIG. 29), respectively. General experimental procedures of the immunofluorescence flow cytometry and confocal microscopy were performed in the same manner as in Experimental example 5, and general experimental procedures of the western blotting were performed in the same manner as in Experimental example 3, except that rabbit anti-3D8 scFv serum and an alkaline phosphatase-conjugated anti-rabbit antibody were used to detect the 3D8 scFv protein, and 3-bromo-4-chloro-5-indolyl phosphate (BCIP) and nitrobluetetrazolium (NBT) were used as reaction substrate to induce the visualization of the 3D8 scFv protein. As a result, all of the three analysis methods confirmed that the uptake of the 3D8 scFv protein was observed with a significantly low level in the normal cell line, compared to the cancer cell line (FIGS. 27 to 29). This indicates that the difference in the cytotoxicities of 3D8 scFv to the cancer cell line and the normal cell line arises from the difference in the uptake of the 3D8 scFv protein into cells.

[202]

[203] **Experimental example 14: Comparison of expression levels of glypican-3 on cell surfaces of normal hepatocyte and liver cancer cell line**

[204] An expression level of glypican-3 on cell surfaces of a liver cancer cell line (HepG2) and a normal human hepatocyte (BD Bioscience / Cell source information: Female. 10 years, Caucasian) was analyzed using a flow cytometry after each cell was stained with an anti-glypican-3 antibody. As a result, it was confirmed that the glypican-3 was expressed at a high level in the liver cancer cell line, but was not expressed in the normal hepatocyte at all (FIG. 30). Also, it was confirmed that, when the two cell lines were treated with 3D8 scFv protein (2 hours) and stained with an anti-3D8 scFv antibody to compare the uptakes of 3D8 scFv into cells to each other using flow cytometry, the uptake of the 3D8 scFv was observed at a very high level in the HepG2 cell overexpressing glypican-3, but was nearly not observed in the normal hepatocyte (FIG. 31). This indicates that the gypican-3 acts as a receptor in the uptake of the 3D8 scFv cell.

[205]

[206] **Experimental example 15: Comparison of toxicities of 3D8 scFv protein in normal hepatocyte and liver cancer cell line**

[207] In order to determine the toxicity of 3D8 scFv protein in a liver cancer cell line (HepG2) and a normal human hepatocyte (BD Bioscience / Cell source information: Female. 10 years, Caucasian), the normal hepatocyte and the HepG2 liver cancer cell line were treated with 3D8 scFv protein, and incubated at 37°C for 48 hours in a CO₂ incubator. After an MTT solution was added to each of the incubated cell lines, the incubated cell lines were reacted at 37°C for 4 hours in a CO₂ incubator.

[208] Here, a 96-well plate coated with 50 ug/ml of collagen (Rat Tail Collagen, Type 1 : BD bioscience cat. No. 354236) was used for the cell culture. After the 4-hour cell culture, the used culture solution was removed, and DMSO was added to each well and thoroughly mixed to measure absorbance values at 595 nm (FIGS. 32 to 33). As a result, it was confirmed that the cell death was readily induced in the normal hepatocyte (FIG. 33), compared to the HepG2 cell line (FIG. 32). Also, when the genomic DNA was extracted from the HepG2 cell line showing the cytotoxicity in the treatment with the 3D8 scFv, and subjected to agarose gel electrophoresis, the DNA fragmentation was observed, which indicates that the apoptosis is induced by the 3D8 scFv protein (FIG. 34).

[209]

[210] **Experimental example 16: Presence of endotoxin in 3D8 scFv protein purified from bacteria**

[211] Prior to determination of the anti-cancer activity of 3D8 scFv in an *In-vivo* mouse model, the presence of endotoxin contaminated during the purification of 3D8 scFv protein was measured using an endotoxin detection kit [Pyrosate kit cat. no PSD10; Associates of Cape Cod, Inc (www.acciusa.com)]. The endotoxin detection kit has a sensitivity of 0.25 EU/ml, and its operating principle is based on the fact that an indicator is changed into a gel phase when endotoxin is present in a concentration of 0.25 EU/ml or more in protein. The gelation of the indicator did not appear when 1 ug of the purified 3D8 scFv protein reacted with the indicator. Therefore, it was confirmed that 0.25EU/ml or more of the endotoxin is not present in 1 *µ*g of the purified 3D8 scFv protein (FIG. 35).

[212]

[213] **Experimental example 17: observation of anti-cancer activity of 3D8 scFv in mouse model**

[214] In order to determine the cancer cells activity of 3D8 scFv in an animal model, a human liver cancer cell line (HepG2) was subcutaneously injected to right femoral regions of 5-week-old nude mice (5 x 10⁶ cells in 100 *µ*ℓ PBS/mouse). Here, PBS was used as the negative control, and 8 mice were used per group. When a tumor volume of cancer of the 3D8 scFv-injected mouse reached approximately 300 mm³ (9^{th} day), the 3D8 scFv protein (60 *µ*g 3D8 scFv/20g mouse) was injected intratumorly once a day for 3 days. Then, a tumor volume of cancer cell was measured with 3-day intervals for 3 weeks by using a caliper (FIG. 36). As a result, it was confirmed that the cancer cell growth was conspicuously suppressed in the 3D8 scFv protein-administered mouse group, compared to a group of the negative control. Also, it was confirmed that, when cancerous lumps are extracted from the mice, the cancerous lumps are significantly small in volume in the 3D8 scFv protein-administered mouse group, compared to a group of the negative control. (FIG. 37).

[215]

[216] **Experimental example 18: Toxicity of 3D8 scFv to liver cancer cells isolated from liver cancer patient (HCC patient)**

[217] A liver cancer tissue isolated from a liver cancer patient was washed with PBS, cut into small test samples with 1 mm³. Then, the test samples of the liver cancer tissue were treated with 0.1% collagenase type IV (Sigma/cat. no C-5138) at 37°C for 30 minutes, strained through a 40-mm nylon mesh (BD Falcon, cat no. 352340) to remove tissue debris, and then centrifuged (at a rotary speed of 1,000 rpm at 4°C for 5 minutes) to harvest cells. An erythrocyte lysate solution (150mM NH₂Cl, 1mM KHCO₃, 0.1nM EDTA, pH 7.2-7.4) was added to the harvested cells, and kept at 37°C for 5 minutes to get rid of red blood cells. The resulting cells were suspended in an F12 medium (GIBCO BRL, cat no.31765035) supplemented with 20% FBS, and incubated in a 96-well plate coated with 50 *µ*g/ml of collagen (Rat Tail Collagen, Type 1 : BD bioscience cat. No. 354236). Then, the cultured cells were treated with 3D8 scFv for 48 hours, and measured for cell viability using an MTT assay. As a result, it was confirmed that approximately 90% of the liver cancer cells died when the liver cancer cells are treated with the 5uM 3D8 scFv protein (FIG. 38).

[218]
<110> Ajou University Industry Cooperation Foundation
<120> Anti-nucleic acid antibody inducing cell death of cancer cells and composition for preventing or treating cancers comprising the same
<150> KR20080040294
   <151> 2008-04-30
<160> 10
<170> KopatentIn 1.71
<210> 1
   <211> 251
   <212> PRT
   <213> Artificial Sequence
<220>
   <223> 3D8 scFv
<400> 1
   Ala Arg Glu Val Gln Leu Gln Gln Ser Gly Pro Glu Leu Val Lys Pro
    1               5                  10                  15
   Gly Ala Ser Val Lys Met Ser Cys Lys Ala Ser Gly Tyr Thr Phe Thr
               20                  25                  30
   Ser Tyr Val Met His Trp Val Lys Gln Lys Pro Gly Gln Gly Leu Glu
           35                  40                  45
   Trp Ile Gly Tyr Ile Asn Pro Tyr Asn Asp Gly Thr Lys Tyr Asn Glu
       50                  55                  60
   Lys Phe Lys Gly Lys Ala Thr Leu Thr Ser Asp Lys Ser Ser Ser Thr
   65                  70                  75                  80
   Ala Tyr Met Glu Leu Ser Ser Leu Thr Ser Glu Asp Ser Ala Val Tyr
                   85                  90                  95
   Tyr Cys Ala Arg Gly Ala Tyr Lys Arg Gly Tyr Ala Met Asp Tyr Trp
               100                 105                 110
   Gly Gln Gly Thr Ser Val Thr Val Ser Ser Arg Gly Gly Gly Gly Ser
           115                 120                 125
   Gly Gly Gly Gly Ser Gly Gly Gly Gly Ser Asp Leu Val Met Ser Gln
       130                 135                 140
   Ser Pro Ser Ser Leu Ala Val Ser Ala Gly Glu Lys Val Thr Met Ser
   145                 150                 155                 160
   Cys Lys Ser Ser Gln Ser Leu Phe Asn Ser Arg Thr Arg Lys Asn Tyr
                   165                 170                 175
   Leu Ala Trp Tyr Gln Gln Lys Pro Gly Gln Ser Pro Lys Leu Leu Ile
               180                 185                 190
   Tyr Trp Ala Ser Thr Arg Glu Ser Gly Val Pro Asp Arg Phe Thr Gly
           195                 200                 205
   Ser Gly Ser Gly Thr Asp Phe Thr Leu Thr Ile Ser Ser Val Gln Ala
       210                 215                 220
   Glu Asp Leu Ala Val Tyr Tyr Cys Lys Gln Ser Tyr Tyr His Met Tyr
   225                 230                 235                 240
   Thr Phe Gly Ser Gly Thr Lys Leu Glu Ile Lys
                   245                 250
<210> 2
   <211> 122
   <212> PRT
   <213> Artificial Sequence
<220>
   <223> 3D8 VH
<400> 2
   Ala Arg Glu Val Gln Leu Gln Gln Ser Gly Pro Glu Leu Val Lys Pro
    1               5                  10                  15
   Gly Ala Ser Val Lys Met Ser Cys Lys Ala Ser Gly Tyr Thr Phe Thr
               20                  25                  30
   Ser Tyr Val Met His Trp Val Lys Gln Lys Pro Gly Gln Gly Leu Glu
           35                  40                  45
   Trp Ile Gly Tyr Ile Asn Pro Tyr Asn Asp Gly Thr Lys Tyr Asn Glu
       50                  55                  60
   Lys Phe Lys Gly Lys Ala Thr Leu Thr Ser Asp Lys Ser Ser Ser Thr
   65                  70                  75                  80
   Ala Tyr Met Glu Leu Ser Ser Leu Thr Ser Glu Asp Ser Ala Val Tyr
                   85                  90                  95
   Tyr Cys Ala Arg Gly Ala Tyr Lys Arg Gly Tyr Ala Met Asp Tyr Trp
               100                 105                 110
   Gly Gln Gly Thr Ser Val Thr Val Ser Ser
           115                 120
<210> 3
   <211> 113
   <212> PRT
   <213> Artificial Sequence
<220>
   <223> 3D8 VL
<400> 3
   Asp Leu Val Met Ser Gln Ser Pro Ser Ser Leu Ala Val Ser Ala Gly
    1               5                  10                  15
   Glu Lys Val Thr Met Ser Cys Lys Ser Ser Gln Ser Leu Phe Asn Ser
               20                  25                  30
   Arg Thr Arg Lys Asn Tyr Leu Ala Trp Tyr Gln Gln Lys Pro Gly Gln
               35                  40                  45
   Ser Pro Lys Leu Leu Ile Tyr Trp Ala Ser Thr Arg Glu Ser Gly Val
       50                  55                  60
   Pro Asp Arg Phe Thr Gly Ser Gly Ser Gly Thr Asp Phe Thr Leu Thr
   65                  70                  75                  80
   Ile Ser Ser Val Gln Ala Glu Asp Leu Ala Val Tyr Tyr Cys Lys Gln
                   85                  90                  95
   Ser Tyr Tyr His Met Tyr Thr Phe Gly Ser Gly Thr Lys Leu Glu Ile
               100                 105                 110
   Lys
<210> 4
   <211> 753
   <212> DNA
   <213> Artificial Sequence
<220>
   <223> 3D8 scFv
<400> 4
   gcccgggagg tccagctgca gcagtctgga cctgagctgg taaagcctgg ggcttcagtg        60
   aagatgtcct gcaaggcttc tggatacaca ttcactagct atgttatgca ctgggtgaag       120
   cagaagcctg ggcagggcct tgagtggatt ggatatatta atccttacaa tgatggtact       180
   aagtacaatg agaagttcaa aggcaaggcc acactgactt cagacaaatc ctccagcaca       240
   gcctacatgg agctcagcag cctgacctct gaggactctg cggtctatta ctgtgcaaga       300
   ggggcctata aaaggggata tgctatggac tactggggtc aaggaacctc agtcaccgtc       360
   tcctctagag gtgggggcgg ttcgggtggc gggggctcgg gcgggggtgg ctcagatctt       420
   gtgatgtcac agtctccatc ctccctggct gtgtcagcag gagagaaggt cactatgagc       480
   tgcaaatcca gtcagagtct gttcaacagt agaacccgaa agaactactt ggcttggtac       540
   cagcagaaac cagggcagtc tcctaaactg ctgatctact gggcatccac tagggaatct       600
   ggggtccctg atcgcttcac aggcagtgga tctgggacag atttcactct caccatcagc       660
   agtgtgcagg ctgaagacct ggcagtttat tactgcaagc aatcttatta tcacatgtat       720
   acgttcggat cggggaccaa gctggaaata aaa                                    753
<210> 5
   <211> 366
   <212> DNA
   <213> Artificial Sequence
<220>
   <223> 3D8 VH
<40 0> 5
<210> 6
   <211> 339
   <212> DNA
   <213> Artificial Sequence
<220>
   <223> 3D8 VL
<400> 6
   gatcttgtga tgtcacagtc tccatcctcc ctggctgtgt cagcaggaga gaaggtcact 60
   atgagctgca aatccagtca gagtctgttc aacagtagaa cccgaaagaa ctacttggct 120
   tggtaccagc agaaaccagg gcagtctcct aaactgctga tctactgggc atccactagg 180
   gaatctgggg tccctgatcg cttcacaggc agtggatctg ggacagattt cactctcacc 240
   atcagcagtg tgcaggctga agacctggca gtttattact gcaagcaatc ttattatcac 300
   atgtatacgt tcggatcggg gaccaagctg gaaataaaa 339
<210> 7
   <211> 26
   <212> DNA
   <213> Artificial Sequence
<220>
   <223> Primer for VH
<400> 7
   atgggatgga gctrtatcat sytctt 26
<210> 8
   <211> 30
   <212> DNA
   <213> Artificial Sequence
<220>
   <223> Primer for VH
<400> 8
   tggatagaca gatgggggtg tcgttttggc 30
<210> 9
   <211> 30
   <212> DNA
   <213> Artificial Sequence
<220>
   <223> Primer for VL
<400> 9
   atgaagttgc ctgttaggct gttgtgtctc 30
<210> 10
   <211> 21
   <212> DNA
   <213> Artificial Sequence
<220>
   <223> Primer for VL
<400> 10
   ggatggtggg aagatggata c 21

## Claims

1. An antibody protein comprising:
(i) a scFv having the sequence set forth in SEQ ID NO: 1; or
(ii) a Fv comprising a VH having the sequence set forth in SEQ ID NO: 2 and a VL having the sequence set forth in SEQ ID NO: 3,
for use in preventing or treating cancer.

2. The antibody protein for use according to claim 1, wherein the cancer is selected from the group consisting of cervix cancer, colon cancer, neuroblastoma and liver cancer.

## Patentansprüche

1. Antikörperprotein, umfassend:
(i) ein scFv mit der Sequenz, die in SEQ ID NR: 1 dargelegt ist; oder
(ii) ein Fv, umfassend ein VH mit der Sequenz, die in SEQ ID NR: 2 dargelegt ist, und ein VL mit der Sequenz, die in SEQ ID NR: 3 dargelegt ist,
zur Benutzung zum Vorbeugen gegen oder Behandeln von Krebs.

2. Antikörperprotein zur Benutzung nach Anspruch 1, wobei der Krebs aus der Gruppe ausgewählt ist, die aus Gebärmutterhalskrebs, Darmkrebs, Neuroblastom und Leberkrebs besteht.

## Revendications

1. Une protéine anticorps comprenant:
(i) une scFv ayant la séquence décrite dans SEQ ID NO: 1, ou
(ii) une Fv comprenant une VH ayant la séquence décrite dans SEQ ID NO:2 et une VL ayant la séquence décrite dans SEQ ID NO:3
pour son utilisation dans la prévention ou le traitement du cancer.

2. Une protéine anticorps pour son utilisation selon la revendication 1, dans laquelle le cancer est sélectioné parmi le groupe consistant en le cancer du col de l'utérus (cervix cancer), le cancer du colon, les neuoblastomes et le cancer du foie.
